(19) Europäisches Patentamt
European Patent Office
Office européen des brevets

(11) EP 1 801 592 B1

(12) EUROPEAN PATENT SPECIFICATION

(45) Date of publication and mention of the grant of the patent:
**31.12.2008 Bulletin 2009/01**

(21) Application number: **06110392.5**

(22) Date of filing: **24.02.2006**

(51) Int Cl.:
***G01N 33/576*** *(2006.01)* ***C07K 16/10*** *(2006.01)*
***A61K 47/42*** *(2006.01)* ***A61K 47/48*** *(2006.01)*
***A61K 39/00*** *(2006.01)*

(54) **Hepatitis C virus NS2/3 assay**

Hepatitis C Virus NS2/3 Assay

Test du virus NS2/3 de l'hépatite C

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.12.2005 US 275284**

(43) Date of publication of application:
**27.06.2007 Bulletin 2007/26**

(73) Proprietors:
- **Boehringer Ingelheim International GmbH**
**55216 Ingelheim am Rhein (DE)**
- **Boehringer Ingelheim Pharma GmbH & Co.KG**
**55216 Ingelheim am Rhein (DE)**

(72) Inventors:
- **Lamarre, Lyne**
**Laval**
**Québec H7S2G5 (CA)**
- **Lagace, Lisette**
**Laval**
**Québec H7S2G5 (CA)**
- **Thibeault, Diane**
**Laval**
**Québec H7S2G5 (CA)**

(74) Representative: **Hammann, Heinz et al**
**Boehringer Ingelheim Pharma GmbH & Co. KG**
**Binger Strasse 173**
**55216 Ingelheim am Rhein (DE)**

(56) References cited:
**US-A1- 2002 192 640**

- **THIBEAULT D ET AL: "In vitro characterization of a purified NS2/3 protease variant of hepatitis C virus" JOURNAL OF BIOLOGICAL CHEMISTRY, AMERICAN SOCIETY OF BIOLOGICHEMICAL BIOLOGISTS, BIRMINGHAM,, US, vol. 276, no. 49, 7 December 2001 (2001-12-07), pages 46678-46684, XP002221840 ISSN: 0021-9258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 801 592 B1

**SEQUENCE LISTING**

<110> Boehringer Ingelheim International GmbH

<120> Hepatitis C Virus NS2/3 Assay

<130> 13-138 EP

<160> 17

<170> FastSEQ for Windows Version 4.0

<210> 1
<211> 1191
<212> DNA
<213> HCV

<220>
<221> CDS
<222> (1) ... (1191)
<223> Nucleotide sequence of NS2/3

<400> 1

```
atg gac cgg gag atg gct gca tcg tgc gga ggc gcg gtt ttc ata ggt     48
Met Asp Arg Glu Met Ala Ala Ser Cys Gly Gly Ala Val Phe Ile Gly
 1               5                  10                  15

ctt gca ctc ttg acc ttg tca cca tac tat aaa gtg ctc ctc gct agg     96
Leu Ala Leu Leu Thr Leu Ser Pro Tyr Tyr Lys Val Leu Leu Ala Arg
            20                  25                  30

ctc ata tgg tgg tta cag tat tta atc acc aga gtc gag gcg cac ttg    144
Leu Ile Trp Trp Leu Gln Tyr Leu Ile Thr Arg Val Glu Ala His Leu
        35                  40                  45

caa gtg tgg atc ccc cct ctc aat gtt cgg gga ggc cgc gat gcc atc    192
Gln Val Trp Ile Pro Pro Leu Asn Val Arg Gly Gly Arg Asp Ala Ile
    50                  55                  60

atc ctc ctc acg tgc gca gtc cac cca gag cta atc ttt gac atc acc    240
Ile Leu Leu Thr Cys Ala Val His Pro Glu Leu Ile Phe Asp Ile Thr
65                  70                  75                  80

aaa ctc ctg ctc gcc ata ttc ggt ccg ctc atg gtg ctc cag gca ggc    288
Lys Leu Leu Leu Ala Ile Phe Gly Pro Leu Met Val Leu Gln Ala Gly
                85                  90                  95

ata acc aaa gtg ccg tac ttc gtg cgt gcg cag ggg ctc att cgt gcg    336
Ile Thr Lys Val Pro Tyr Phe Val Arg Ala Gln Gly Leu Ile Arg Ala
            100                 105                 110

tgt atg ttg gtg cgg aag gct gcg ggg ggt cat tat gtc caa atg gcc    384
Cys Met Leu Val Arg Lys Ala Ala Gly Gly His Tyr Val Gln Met Ala
        115                 120                 125

ttc atg aag cta gct gcg ctg aca ggt acg tac gtt tat gac cat ctc    432
Phe Met Lys Leu Ala Ala Leu Thr Gly Thr Tyr Val Tyr Asp His Leu
    130                 135                 140
```

```
act cca ttg cag gat tgg gcc cac gcg ggc cta cga gac ctt gca gtg    480
Thr Pro Leu Gln Asp Trp Ala His Ala Gly Leu Arg Asp Leu Ala Val
145                 150                 155                 160

gcg gta gag ccc gtc atc ttc tct gac atg gag gtc aag atc atc acc    528
Ala Val Glu Pro Val Ile Phe Ser Asp Met Glu Val Lys Ile Ile Thr
                165                 170                 175

tgg ggg gcg gac acc gcg gca tgc ggg gac atc att tca ggt ctg ccc    576
Trp Gly Ala Asp Thr Ala Ala Cys Gly Asp Ile Ile Ser Gly Leu Pro
            180                 185                 190

gtc tcc gct cga agg gga agg gag ata ctc ctg gga ccg gcc gat aat    624
Val Ser Ala Arg Arg Gly Arg Glu Ile Leu Leu Gly Pro Ala Asp Asn
        195                 200                 205

ttt gaa ggg cag ggg tgg cga ctc ctt gcg ccc atc acg gcc tac tcc    672
Phe Glu Gly Gln Gly Trp Arg Leu Leu Ala Pro Ile Thr Ala Tyr Ser
    210                 215                 220

caa cag aca cgg ggc cta ctt ggt tgc atc atc acc agc ctc aca ggc    720
Gln Gln Thr Arg Gly Leu Leu Gly Cys Ile Ile Thr Ser Leu Thr Gly
225                 230                 235                 240

cgg gac aag aac cag gtc gag ggg gag gtt caa gtg gtc tcc acc gct    768
Arg Asp Lys Asn Gln Val Glu Gly Glu Val Gln Val Val Ser Thr Ala
                245                 250                 255

aca caa tct ttc ctg gcg acc tgc gtc aac ggc gtg tgt tgg act gtc    816
Thr Gln Ser Phe Leu Ala Thr Cys Val Asn Gly Val Cys Trp Thr Val
            260                 265                 270

ttc cat ggc gcc ggc tca aag acc ttg gcc ggc ccc aaa ggc cca atc    864
Phe His Gly Ala Gly Ser Lys Thr Leu Ala Gly Pro Lys Gly Pro Ile
        275                 280                 285

acc cag atg tac act aat gtg gac cag gac ctc gtc ggc tgg cag gcg    912
Thr Gln Met Tyr Thr Asn Val Asp Gln Asp Leu Val Gly Trp Gln Ala
    290                 295                 300

ccc cct ggg gcg cgc tcc atg aca cca tgc acc tgc ggc agc tcg gac    960
Pro Pro Gly Ala Arg Ser Met Thr Pro Cys Thr Cys Gly Ser Ser Asp
305                 310                 315                 320

ctc tat ttg gtc acg aga cat gcc gac gtc att ccg gtg cgc cgg cgg   1008
Leu Tyr Leu Val Thr Arg His Ala Asp Val Ile Pro Val Arg Arg Arg
                325                 330                 335

ggc gac agt agg ggg agc ctg ctc tcc ccc agg cct gtc tcc tac ttg   1056
Gly Asp Ser Arg Gly Ser Leu Leu Ser Pro Arg Pro Val Ser Tyr Leu
            340                 345                 350

aag ggc tct tcg ggt ggc cca ctg ctc tgc cct tcg ggg cac gtt gtg   1104
Lys Gly Ser Ser Gly Gly Pro Leu Leu Cys Pro Ser Gly His Val Val
        355                 360                 365

ggc atc ttc cgg gct gct gtg tgc acc cgg ggg gtt gca aaa gcg gtg   1152
Gly Ile Phe Arg Ala Ala Val Cys Thr Arg Gly Val Ala Lys Ala Val
    370                 375                 380

gac ttc ata cct gtt gag tct atg gaa act acc atg cgg               1191
```

```
Asp Phe Ile Pro Val Glu Ser Met Glu Thr Thr Met Arg
385                 390                 395
```

<210> 2
<211> 300
<212> PRT
<213> HCV

<400> 2

```
Thr Lys Val Pro Tyr Phe Val Arg Ala Gln Gly Leu Ile Arg Ala Cys
1               5                   10                  15
Met Leu Val Arg Lys Ala Ala Gly Gly His Tyr Val Gln Met Ala Phe
            20                  25                  30
Met Lys Leu Ala Ala Leu Thr Gly Thr Tyr Val Tyr Asp His Leu Thr
        35                  40                  45
Pro Leu Gln Asp Trp Ala His Ala Gly Leu Arg Asp Leu Ala Val Ala
    50                  55                  60
Val Glu Pro Val Ile Phe Ser Asp Met Glu Val Lys Ile Ile Thr Trp
65                  70                  75                  80
Gly Ala Asp Thr Ala Ala Cys Gly Asp Ile Ile Ser Gly Leu Pro Val
                85                  90                  95
Ser Ala Arg Arg Gly Arg Glu Ile Leu Leu Gly Pro Ala Asp Asn Phe
            100                 105                 110
Glu Gly Gln Gly Trp Arg Leu Leu Ala Pro Ile Thr Ala Tyr Ser Gln
        115                 120                 125
Gln Thr Arg Gly Leu Leu Gly Cys Ile Ile Thr Ser Leu Thr Gly Arg
    130                 135                 140
Asp Lys Asn Gln Val Glu Gly Glu Val Gln Val Val Ser Thr Ala Thr
145                 150                 155                 160
Gln Ser Phe Leu Ala Thr Cys Val Asn Gly Val Cys Trp Thr Val Phe
                165                 170                 175
His Gly Ala Gly Ser Lys Thr Leu Ala Gly Pro Lys Gly Pro Ile Thr
            180                 185                 190
Gln Met Tyr Thr Asn Val Asp Gln Asp Leu Val Gly Trp Gln Ala Pro
        195                 200                 205
Pro Gly Ala Arg Ser Met Thr Pro Cys Thr Cys Gly Ser Ser Asp Leu
    210                 215                 220
Tyr Leu Val Thr Arg His Ala Asp Val Ile Pro Val Arg Arg Arg Gly
225                 230                 235                 240
Asp Ser Arg Gly Ser Leu Leu Ser Pro Arg Pro Val Ser Tyr Leu Lys
                245                 250                 255
Gly Ser Ser Gly Gly Pro Leu Leu Cys Pro Ser Gly His Val Val Gly
            260                 265                 270
Ile Phe Arg Ala Ala Val Cys Thr Arg Gly Val Ala Lys Ala Val Asp
        275                 280                 285
Phe Ile Pro Val Glu Ser Met Glu Thr Thr Met Arg
    290                 295                 300
```

<210> 3
<211> 303
<212> PRT
<213> HCV

<400> 3

```
Ala Gly Ile Thr Lys Val Pro Tyr Phe Val Arg Ala Gln Gly Leu Ile
1               5                   10                  15
Arg Ala Cys Met Leu Val Arg Lys Ala Ala Gly Gly His Tyr Val Gln
            20                  25                  30
Met Ala Phe Met Lys Leu Ala Ala Leu Thr Gly Thr Tyr Val Tyr Asp
        35                  40                  45

His Leu Thr Pro Leu Gln Asp Trp Ala His Ala Gly Leu Arg Asp Leu
    50                  55                  60
Ala Val Ala Val Glu Pro Val Ile Phe Ser Asp Met Glu Val Lys Ile
65                  70                  75                  80
Ile Thr Trp Gly Ala Asp Thr Ala Ala Cys Gly Asp Ile Ile Ser Gly
                85                  90                  95
Leu Pro Val Ser Ala Arg Arg Gly Arg Glu Ile Leu Leu Gly Pro Ala
            100                 105                 110
Asp Asn Phe Glu Gly Gln Gly Trp Arg Leu Leu Ala Pro Ile Thr Ala
        115                 120                 125
Tyr Ser Gln Gln Thr Arg Gly Leu Leu Gly Cys Ile Ile Thr Ser Leu
    130                 135                 140
Thr Gly Arg Asp Lys Asn Gln Val Glu Gly Glu Val Gln Val Val Ser
145                 150                 155                 160
Thr Ala Thr Gln Ser Phe Leu Ala Thr Cys Val Asn Gly Val Cys Trp
                165                 170                 175
Thr Val Phe His Gly Ala Gly Ser Lys Thr Leu Ala Gly Pro Lys Gly
            180                 185                 190
Pro Ile Thr Gln Met Tyr Thr Asn Val Asp Gln Asp Leu Val Gly Trp
        195                 200                 205
Gln Ala Pro Pro Gly Ala Arg Ser Met Thr Pro Cys Thr Cys Gly Ser
    210                 215                 220
Ser Asp Leu Tyr Leu Val Thr Arg His Ala Asp Val Ile Pro Val Arg
225                 230                 235                 240
Arg Arg Gly Asp Ser Arg Gly Ser Leu Leu Ser Pro Arg Pro Val Ser
                245                 250                 255
Tyr Leu Lys Gly Ser Ser Gly Gly Pro Leu Leu Cys Pro Ser Gly His
            260                 265                 270
Val Val Gly Ile Phe Arg Ala Ala Val Cys Thr Arg Gly Val Ala Lys
        275                 280                 285
Ala Val Asp Phe Ile Pro Val Glu Ser Met Glu Thr Thr Met Arg
    290                 295                 300
```

<210> 4
<211> 334
<212> PRT
<213> HCV

<400> 4

```
Met Lys Lys Lys Lys Leu Glu His His His His His His Thr Ser Ala
1               5                   10                  15
Gly Ile Thr Lys Val Pro Tyr Phe Val Arg Ala Gln Gly Leu Ile Arg
            20                  25                  30
Ala Cys Met Leu Val Arg Lys Ala Ala Gly Gly His Tyr Val Gln Met
        35                  40                  45
Ala Phe Met Lys Leu Ala Ala Leu Thr Gly Thr Tyr Val Tyr Asp His
    50                  55                  60
Leu Thr Pro Leu Gln Asp Trp Ala His Ala Gly Leu Arg Asp Leu Ala
65                  70                  75                  80
Val Ala Val Glu Pro Val Ile Phe Ser Asp Met Glu Val Lys Ile Ile
                85                  90                  95
Thr Trp Gly Ala Asp Thr Ala Ala Cys Gly Asp Ile Ile Ser Gly Leu
            100                 105                 110
Pro Val Ser Ala Arg Arg Gly Arg Glu Ile Leu Leu Gly Pro Ala Asp
        115                 120                 125
Asn Phe Glu Gly Gln Gly Trp Arg Leu Leu Ala Pro Ile Thr Ala Tyr
    130                 135                 140
Ser Gln Gln Thr Arg Gly Leu Leu Gly Cys Ile Ile Thr Ser Leu Thr
145                 150                 155                 160
Gly Arg Asp Lys Asn Gln Val Glu Gly Glu Val Gln Val Val Ser Thr
                165                 170                 175

Ala Thr Gln Ser Phe Leu Ala Thr Cys Val Asn Gly Val Cys Trp Thr
            180                 185                 190
Val Phe His Gly Ala Gly Ser Lys Thr Leu Ala Gly Pro Lys Gly Pro
        195                 200                 205
Ile Thr Gln Met Tyr Thr Asn Val Asp Gln Asp Leu Val Gly Trp Gln
    210                 215                 220
Ala Pro Pro Gly Ala Arg Ser Met Thr Pro Cys Thr Cys Gly Ser Ser
225                 230                 235                 240
Asp Leu Tyr Leu Val Thr Arg His Ala Asp Val Ile Pro Val Arg Arg
                245                 250                 255
Arg Gly Asp Ser Arg Gly Ser Leu Leu Ser Pro Arg Pro Val Ser Tyr
            260                 265                 270
Leu Lys Gly Ser Ser Gly Gly Pro Leu Leu Cys Pro Ser Gly His Val
        275                 280                 285
Val Gly Ile Phe Arg Ala Ala Val Cys Thr Arg Gly Val Ala Lys Ala
    290                 295                 300
Val Asp Phe Ile Pro Val Glu Ser Met Glu Thr Thr Met Arg Thr Ser
305                 310                 315                 320
Ser Ala Trp Arg His Pro Gln Phe Gly Gly Lys Lys Lys Lys.
                325                 330
```

<210> 5
<211> 10
<212> PRT
<213> hcv

<400> 5

```
Ala Pro Ile Thr Ala Tyr Ser Gln Gln Thr
1               5                   10
```

<210> 6
<211> 10
<212> PRT
<213> hcv

<400> 6

Ser Phe Glu Gly Gln Gly Trp Arg Leu Leu
1 5 10

<210> 7
<211> 17
<212> PRT
<213> hcv

<400> 7

Lys Lys Gly Ser Val Val Ile Val Gly Arg Ile Ile Leu Ser Gly Arg
1 5 10 15
Lys

<210> 8
<211> 10
<212> PRT
<213> HCV

<400> 8

Asn Phe Glu Gly Gln Gly Trp Arg Leu Leu
1 5 10

<210> 9
<211> 10
<212> PRT
<213> HCV

<400> 9

Asp Asn Phe Glu Gly Gln Gly Trp Arg Leu
1 5 10

<210> 10
<211> 10
<212> PRT
<213> HCV

<400> 10

Ala Asp Asn Phe Glu Gly Gln Gly Trp Arg
1 5 10

<210> 11
<211> 10
<212> PRT
<213> HCV

<400> 11

Pro Ala Asp Asn Phe Glu Gly Gln Gly Trp
1 5 10

<210> 12
<211> 10
<212> PRT
<213> HCV

<400> 12

Gly Pro Ala Asp Asn Phe Glu Gly Gln Gly
1 5 10

<210> 13
<211> 10
<212> PRT
<213> HCV

<400> 13

Ser Ala Arg Arg Gly Arg Glu Ile Leu Leu
1 5 10

<210> 14
<211> 10
<212> PRT
<213> HCV

<400> 14

Pro Ile Thr Ala Tyr Ser Gln Gln Thr Thr
1 5 10

<210> 15
<211> 10
<212> PRT
<213> HCV

<400> 15

Ile Thr Ala Tyr Ser Gln Gln Thr Thr Arg
1 5 10

<210> 16
<211> 10
<212> PRT
<213> HCV

<400> 16

Thr Ala Tyr Ser Gln Gln Thr Thr Arg Gly
1 5 10

<210> 17
<211> 10
<212> PRT
<213> HCV

<400> 17

```
Arg Gly Leu Leu Gly Cys Ile Ile Thr Ser
1               5                   10
```

## Description

### FIELD OF THE INVENTION

The present invention relates to an assay for detecting cleavage of HCV protein in a sample, and more particularly, to an assay for the selective detection of HCV NS2/3 autocleavage activity, and even more particularly to the identification of potential HCV inhibitor compounds.

### BACKGROUND OF THE INVENTION

Hepatitis C virus (HCV) is the major etiological agent of post-transfusion and community-acquired non-A non-B hepatitis worldwide. A high percentage of carriers become chronically infected and many progress to chronic liver disease, so called chronic hepatitis C. This group is in turn at high risk for serious liver disease such as liver cirrhosis, hepatocellular carcinoma and terminal liver disease leading to death.

HCV is an enveloped positive strand RNA virus in the *Flaviviridae* family. The single strand HCV RNA genome is of positive polarity and comprises one open reading frame (ORF) of approximately 9600 nucleotides in length, which encodes a linear polyprotein of approx. 3010 amino acids. In infected cells, this polyprotein is cleaved at multiple sites by cellular and viral proteases to produce structural and non-structural (NS) proteins. The structural proteins (C, E1, E2 and p7) comprise polypeptides that constitute the viral particle. Processing of the structural proteins is catalyzed by host cell proteases. The non-structural proteins (NS2, NS3, NS4A, NS4B, NS5A, NS5B) encode for enzymes or accessory factors that catalyze and regulate the replication of the HCV RNA genome. The generation of the mature non-structural proteins is catalyzed by two virally encoded proteases. The first is the NS2/3 protease which auto-catalyses the cleavage between NS2 and NS3. The NS3 contains a N-terminal serine protease domain and catalyzes the remaining cleavages from the polyprotein. The released NS4A protein has at least two roles. The first role is forming a stable complex with NS3 protein and assisting in the membrane localization of the NS3/NS4A complex; the second is acting as a cofactor for NS3 protease activity. This membrane-associated complex, in turn catalyzes the cleavage of the remaining sites on the polyprotein, thus effecting the release of NS4B, NS5A and NS5B.

The cleavage of the Hepatitis C Virus (HCV) polyprotein between the nonstructural proteins NS2 and NS3 is mediated by the NS2/3 protease, a protease activity that is encoded by the NS2 region and the minimal NS3 protease domain which flank the cleavage site. NS2/3 protease is expressed in virally infected hepatocytes and experimental data are consistent with its essential role in viral propagation and disease. Indeed, no productive infection was observed in chimpanzees upon inoculation of HCV clones containing mutations abolishing NS2/3 protease activity, suggesting that this HCV-encoded enzyme is essential for productive replication *in vivo* (**1**).

A minimal catalytic region of NS2/3 protease has been defined and includes the C-terminus of NS2 and the N-terminal NS3 protease domain (**2-5**). The NS2/3 (904-1206) variant from HCV genotype 1 b was purified from *E. coli* inclusion bodies and refolded by gel filtration chromatography as previously described (**2, 3**). The purified inactive form of NS2/3 (904-1206) can be activated by the addition of glycerol and detergent to induce autocleavage at the predicted site between the residues leucine 1026 and alanine 1027 (**2, 3**).

NS2/3 protease cleavage detection assays based on the separation of the NS2 and NS3 products from the NS2/3 precursor by SDS-PAGE and by HPLC have been reported, as well as an assay based on the NS3 protease activity of the NS2/3 protein which also requires separation of the NS2/3 uncleaved precursor from the NS3 protease product (**2-5**). Such methods can be time-consuming and are not adapted for rapid screening. Moreover, no assay has yet been developed having the selectivity to detect NS2/3 cleavage products in the presence of uncleaved NS2/3.

It would, thus, be desirable to develop efficient NS2/3 cleavage assays which overcome one or more disadvantages of existing assays.

Novel selective assay methods are provided comprising cleavage of NS2/3 protease in a sample and treatment of the cleaved sample which enables detection of cleavage products NS2 or NS3 therein.

### SUMMARY OF THE INVENTION

The present invention provides a novel assay for NS2/3 cleavage detection. More particularly, the present invention provides a novel assay for the detection of the NS2/3 cleavage products NS2 or NS3 in the presence of uncleaved NS2/3.

In the present invention, following self-cleavage of NS2/3 to generate NS2 and NS3 cleavage products, the sample is incubated with a ligand specific for the recognition of NS2 or NS3 cleavage product in the presence of uncleaved NS2/3.

In a first aspect of the present invention, there is provided a method for detecting a NS2/3 autocleavage product in a sample containing refolded, inactive NS2/3 protease, the method comprising:

a) subjecting the sample to suitable conditions under which at least a portion of the NS2/3 protease is self-cleaved to yield cleaved NS2 and NS3 products;
b) incubating the NS2 and NS3 products with a ligand that can preferentially bind to one of either NS2 or NS3 product over NS2/3 under conditions suitable to afford binding of the ligand to NS2 or NS3; and
c) detecting NS2- or NS3-bound ligand produced in step b), whereby the amount of bound ligand detected correlates with the NS2/3 autocleavage activity.

The present method is also useful as an assay to screen candidate NS2/3 inhibitor compounds.
A second aspect provides for an assay for screening a candidate compound for NS2/3 cleavage inhibitory activity in a sample containing refolded, inactive NS2/3 protease, the assay comprising:

a) subjecting a first sample comprising NS2/3 protease to suitable conditions under which at least a portion of NS2/3 is self-cleaved to yield cleaved NS2 and NS3 products in the absence of a candidate compound;
b) subjecting a second sample comprising NS2/3 protease in the presence of a candidate compound under the same conditions as those in step a);
c) incubating the first and second samples with a ligand that preferentially binds to one of either NS2 or NS3 product over NS2/3 under conditions suitable to cause binding of the ligand to NS2 or NS3; and
d) determining the amount of ligand bound to the second sample and to the first sample produced in step c), whereby a decrease in the amount of ligand in the second sample compared to that of the first sample indicates that the candidate compound may be an inhibitor of NS2/3 autocleavage activity.

A further aspect of the present invention concerns ligands selectively recognizing one of the NS2 cleaved product or the NS3 cleaved product with minimal cross-reactivity with the uncleaved NS2/3 and the other cleaved product. Specifically, the present invention provides antibodies that selectively recognize cleaved NS3 product with minimal cross-reactivity with the uncleaved NS2/3 and the NS2 cleaved product. Alternatively, the present invention provides antibodies that selectively recognize cleaved NS2 product with minimal cross-reactivity with the uncleaved NS2/3 and the NS3 cleaved product.
As will be recognized by persons of skill in the art, other types of auto-cleaving proteases similar or homologous to the HCV NS2/3 protease may be used in the method / assay of the present invention in the search for respective inhibitors. Such other proteases may be found in pestiviruses such as, but not limited to: GB virus A, B, or C; bovine viral diarrhea virus (BVDV); Classical Swine Fever virus; Border disease virus; bovine pestivirus; and porcine pestivirus.
These and other aspects of the present invention are described herein by reference to the following figures.

## BRIEF DESCRIPTION OF THE DRAWINGS

**Figure 1A** is a schematic representation of an NS2/3 protease assay in accordance with one aspect of the present invention;

**Figure 1B** is a schematic representation of an NS2/3 protease assay in accordance with another aspect of the present invention;

**Figure 2A** illustrates the dilution curve of the K147 polyclonal antibody as determined by ELISA assay;

**Figure 2B** is a Western blot demonstrating the selective binding of K147 antibody to (+) cleaved NS3 product compared to (-) uncleaved NS2/3;

**Figure 3** graphically illustrates the correlation between binding of K147 antibody to the autocleavage activity of the NS2/3 protease according to the method of Example 2;

**Figure 4** graphically illustrates the concentration of NS2/3 protease in the cleavage step according to the method of Example 3;

**Figure 5** graphically illustrates the time dependence of NS2/3 protease cleavage step according to the method of Example 3;

**Figure 6** graphically illustrates the titration of the NS3-selective rabbit polyclonal antibody K147;

**Figure 7A** is a schematic representation of an NS2/3 protease assay in accordance with a specific embodiment of the present invention;

**Figure 7B** is a schematic representation of an NS2/3 protease assay in accordance with another embodiment of the present invention;

**Figure 8** graphically illustrates the Z' obtained with an embodiment of the present NS2/3 protease assay in accordance with Example 5; and

**Figure 9** graphically illustrates an $IC_{50}$ curve of compound **A** obtained with the NS2/3 protease assay in accordance with Example 5.

## DETAILED DESCRIPTION OF THE PRESENT INVENTION

### Definitions

Unless otherwise defined, all technical and scientific terms used herein have the same meaning as those commonly understood by one of ordinary skill in the art to which the invention pertains. Generally, the procedures for cell culture, infection, protein purification, molecular biology methods and the like are common methods used in the art. Such techniques can be found in reference manuals such as, for example, Sambrook et al. (2001, Molecular Cloning - A Laboratory Manual, Cold Spring Harbor Laboratory Press); Ausubel et al. (1994, Current Protocols in Molecular Biology, Wiley, New York) and Coligan et al. (1995, Current Protocols in Protein Science, Volume 1, John Wiley & Sons, Inc., New York).

Nucleotide sequences are presented herein by single strand, in the 5' to 3' direction, from left to right, using the one letter nucleotide symbols as commonly used in the art and in accordance with the recommendations of the IUPAC-IUB Biochemical Nomenclature Commission (Biochemistry, 1972, 11:1726-1732).

All values and concentrations presented herein are subject to inherent variations acceptable in biological science within an error of ± 10%. The term "about" also refers to this acceptable variation.

NS2/3 protease

The term "NS2/3", "NS2/3 protein", "NS2/3 protease" or "uncleaved NS2/3 protease", used herein interchangeably, refer to the region of the Hepatitis C Virus (HCV all genotypes) polyprotein that catalyzes the cleavage of the NS2 domain (810-1026) from the NS3 domain (1027-1615), as well as functionally equivalent variants thereof. In one embodiment as described herein, it is encoded by the native NS2 region (specifically, amino acids 810 to 1026) and the minimal NS3 protease domain (1027 to 1206) of the polyprotein (numbered according to genotype 1a H77 sequence, GenBank accession number AAB67036) herein referred to as 810*-1206 **[SEQ ID NO.1;** *where amino acid 810 corresponds to amino acid 1 of SEQ ID NO.1].

Functionally equivalent variants of the NS2/3 protease are encompassed by the term "NS2/3", "NS2/3 protein", "NS2/3 protease" or "uncleaved NS2/3", such *functionally equivalent* referring to variants able to catalyze the cleavage of NS2/3 such as variants from other HCV isolates/ genotypes. The term "variant" also refers to a protein derived from native NS2/3, but modified in sequence by insertion, deletion, substitution, or modification of one or more amino acids. With respect to amino acid substitutions, these will generally include conservative amino acid substitutions that do not affect the NS2/3 function of the protein as would be appreciated by one of skill in the art. It also includes modified amino acids, for example, amino acids including modified side chains.

Furthermore, a "functionally equivalent variant" refers to truncations comprising the minimal catalytic region of the NS2/3 protease that has been determined to comprise the C-terminus of NS2 (beginning at about amino acid position 907 of the polyprotein) and the N-terminus of NS3 (up to amino acid position 1206) (**5**). Accordingly, NS2/3 truncations comprising these amino acid deletions, termed "NS2/3 fragment" are examples of variants in accordance with the present invention, such as: (907-1206; **SEQ ID NO. 2**) or (904-1206; **SEQ ID NO. 3**). Additionally, NS2/3 deletion mutants comprising any number of amino acid deletions between the native sequence of NS2/3 (810-1615 or 810-1206) and truncated NS2/3 (907-1206) are also contemplated to be variants in accordance with the present invention. Other variants are likewise known in the art, such as those described in WO 01/68818 (**5**), WO 02/48375 & US 6,815,159 (**2**).

As is well recognized within the skill or the art, the term "variant" also encompasses modifications to the protein such as adding affinity tags or detectable labels in order to facilitate extraction/purification or detection/measurement. Also, substitutions or insertions, such as addition of amino acid(s) to enhance solubility (such as lysine), are also encompassed with the term "variant". One example of such variant is ($Lys_4$-$His_6$-904-1206-StrepTag-$Lys_4$) **[SEQ ID NO. 4].**

If a NS2/3 protease functionally equivalent variant is used in the assay in accordance with the present invention, it is necessary to confirm that the modified protein retains NS2/3 autocleavage activity. This can be done using standard cleavage assays such as those described in references **2-5**, cited herein.

The term "at least a portion of NS2/3 protease is cleaved" means that at least a portion of the total amount of the NS2/3 protease present in the assay mixture is cleaved at the 1026-1027 cleavage site.

NS2 product

As used herein, the term "NS2 product" refers to NS2 domain that is cleaved or released from the NS2/3 protease. NS2 product may correspond with native NS2, or may be a functionally equivalent variant thereof. In one embodiment in a construct described herein, native NS2 is represented by amino acids 810-1026 [1-217 of SEQ. ID. No. 1]; however, one of skill in the art will appreciate that NS2 product in accordance with the present invention may be modified by insertion, deletion, modification, substitution of one or more amino acids as described above. It is anticipated that such modifications will correspond with modifications existing in the NS2 portion of the NS2/3 protease utilized in the assay. The term "NS2 product" is interchangeably used herein with the terms "NS2" or "cleaved NS2 product".

Accordingly, NS2 truncations comprising amino acid deletions, termed "NS2 fragment" such as fragments: 907-1026 from SEQ ID NO. 2 or 904-1026 from SEQ ID NO. 3, are examples of variants in accordance with the present invention. Additionally, NS2 deletion mutants comprising any number of amino acid deletions between the native sequence of NS2 (810-1026) and truncated NS2 (907-1026) are also contemplated to be variants in accordance with the present invention.

NS3 product

As used herein, the term " NS3 protease product" refers to NS3 protease domain that is cleaved or released from the NS2/3 protease. NS3 product may correspond with native NS3 (1027-1615), the NS3 protease domain (1027-1206), or may be a functionally equivalent variant thereof, i.e. a variant that retains NS3 protease activity. NS3 product may also correspond to a non-functional variant devoid of NS3 protease activity (such as a S1165A mutant). In one embodiment in a construct described herein, NS3 protease domain is represented by amino acids 1027-1206 [218-397 of SEQ ID NO.1]; however, one of skill in the art will appreciate that NS3 protease product in accordance with the present invention may be modified by insertion, deletion, modification, substitution of one or more amino acids as described above. It is anticipated that such modifications will correspond with modifications existing in the NS3 domain of the NS2/3 protease utilized in the assay. The term "NS3 protease product" is interchangeably used herein with the terms "NS3 product", "NS3 protease" or "cleaved NS3 product".

Accordingly, NS3 truncations comprising amino acid deletions, termed "NS3 fragment" such as fragments: 1027-1187 up to 1027-1205, are examples of variants in accordance with the present invention [NS3 fragment long enough to allow NS2/3 autocleavage but NS3 protease activity is not required]. Additionally, NS3 deletion mutants comprising any number of amino acid deletions between the native sequence of NS3 (1027-1206) and each truncation of NS3 from 1027-1187 up to 1027-1205) (when leading to an active NS2/3 protease) are also contemplated to be variants in accordance with the present invention.

Other useful proteins, enzymes or products of this invention are those linked to an affinity tag to facilitate isolation/separation in the reaction vessel without having to resort to physical separation and transfer to another vessel.

Affinity tag

The term "affinity label" or "affinity tag", as used herein, means a ligand whose affinity for a receptor (or a complementary ligand) can be used to extract (e.g. from a solution) or specifically trap the entity to which the ligand is covalently attached. Affinity tags are indispensable tools that were developed to facilitate the detection and purification of recombinant proteins. They can be classified in two categories: 1) affinity tags that use peptide or protein fusions which bind to small molecule ligands linked to a solid support (hexahistidine tag binding to immobilized transition metals such as nickel, or GST binding to glutathione); or 2) peptide tags binding to an immobilized protein-binding partner (including antibodies) such as the FLAG-tag, the calmodulin-binding peptide, the Strep-tag or Strep-tag II and the biotin acceptor peptide. Examples of pairs of affinity tag/affinity ligand include but are not limited to: Maltose-Binding Protein (MBP)/maltose; Glutathione S Transferase (GST)/ glutathione; histidine (His)/ metal; avidin/ biotin; Strep tag/ streptavidin or neutravidin. The metal used as affinity ligand may be selected from the group consisting of: cobalt, zinc, copper, iron, and nickel. The affinity label may be positioned on the N- or C-terminal end of the protein, or in the middle, but particularly on the N-terminus of the protein. Particularly, the metal selected is nickel. The affinity ligand can be set up in columns to facilitate separation by affinity chromatography. For reference, a review paper was recently published (**6**).

Specific Ligand

The terms "-specific ligand", "-selective ligand", "-directed ligand" or "-preferential ligand" as used herein mean any molecule that binds to another target molecule with specificity. In the context of the present invention a ligand that would bind to the NS2 or NS3 product can be an antibody that has been raised against a specific portion (peptide) of the NS2 or NS3 proteins which has minimal cross-reactivity with other molecules present in the same reaction vessel i.e. the uncleaved NS2/3 protease and the other cleavage product.

The term "antibody" as used herein means an immunoglobulin molecule that has a specific amino acid sequence by virtue of which it interacts selectively with the antigen that induced its synthesis in cells of the lymphoid system or with antigens closely related to it. Such antibodies can be polyclonal or monoclonal, the latter of which is made from a single producing clone.

In particular, the terms "-specific antibody", "-selective antibody", "-directed antibody" or "-preferential antibody" as used herein interchangeably mean that the antibody would yield a signal that is at least about 2 fold higher (i.e. signal window) with its target than with the other cleaved product and the uncleaved NS2/3. More particularly, the selective antibody has a signal window that is about 5 fold or higher. Most particularly, the selective antibody has a signal window that is about 15 fold or higher.

Other useful ligands of this invention are those linked to a detectable label to facilitate detection and measurement.

Detectable label

As used herein, the terms "label", "detectable label" or "detectable marker" refer to any group that may be linked to the specific ligand to allow recognition either directly or indirectly of the resulting ligand-bound molecule such that it can be detected, measured and quantified. Examples of such "labels" include, but are not limited to, fluorescent labels, chemiluminescent labels, colorimetric labels, enzymatic markers, radioactive isotopes and affinity tags such as biotin. Such labels are attached to the peptide or antibody by well known methods. A label, or multiple labels, of the present invention can be introduced at any position on the peptide, for example, the label can be at either the C- or N-terminus or within the peptide or antibody, so long as it does not disturb its functional property of recognizing its specific target molecule.

Practical and useful detectable labels are radioactive labels such as $^{125}I$, fluorescent labels such as fluorescein or lanthanide-complex (i.e. $Eu^{+3}$), or colorimetric labels such as horseradish peroxidase or β-galactosidase and their respective substrate. Such other detectable labels may be found in the Invitrogen- Molecular Probes Handbook - A Guide to Fluorescent Probes and Labeling Technology, 10th ed. 2005 or A guide to HTS Assay Development, D&MD publications ed. April 2004 (and references therein).

As used herein, the term "detergent" means an amphipathic, surface active molecule with polar and non-polar domains. They bind strongly to hydrophobic molecules or molecular domains to confer water solubility. Examples of detergents include, but are not limited to: sodium dodecyl sulphate (SDS), fatty acid salts, the Triton® family, octyl glycoside, 3-[(3-cholamidopropyl)dimethyl-ammonio]-1-propanesulfonate (CHAPS), sodium dodecyl maltoside (DM), lauryldiethylamine oxide (LDAO), NP-40 and the Tween® family.

As used herein, the term "inhibit" or "inhibitor", when used in reference to the NS2/3 protease, is intended to mean that the protease's ability to autocleave is decreased. Drugs or ligands that can inhibit NS2/3 protease (hereinafter referred to as potential "inhibitors") may be useful for modulating HCV infection in a population of cells and, therefore, may be useful as medicaments for treating a pathology characterized by the presence of HCV in the cells.

**Preferred embodiments**

In a particular embodiment of the present invention, there is also provided a method for detecting NS3 product in a sample containing NS2/3 protease, comprising:

a) subjecting the sample to suitable conditions under which at least a portion of the NS2/3 protease is cleaved to yield cleaved NS2 and NS3 products;
a') diluting the protease in said sample to achieve conditions suitable to stop auto-cleavage;
a") immobilizing the NS3 product from step a');
b) incubating the immobilized NS3 product of step a") with a ligand directly or indirectly labeled with a detectable marker wherein said ligand preferentially binds to the NS3 over NS2/3;
c) detecting the immobilized NS3-bound ligand produced in step b), whereby the amount of ligand detected correlates with the NS2/3 autocleavage activity.

In a further embodiment of the present invention, there is also provided a method of detecting NS2 product in a sample containing NS2/3 protease, comprising:

a) subjecting the sample to suitable conditions under which at least a portion of the NS2/3 protease is cleaved to yield cleaved NS2 and NS3 products;
a') diluting the protease in said sample to achieve conditions suitable to stop auto-cleavage;
a") immobilizing the NS2 product from step a');
b) incubating the immobilized NS2 product of step a") with a ligand directly or indirectly labeled with a detectable marker, wherein said ligand preferentially binds to the NS2 over NS2/3;
c) detecting the NS2-bound ligand produced in step b), whereby the amount of ligand detected correlates with the NS2/3 autocleavage activity.

The methods of the present invention are useful as screening assays to identify candidate drug compounds that have NS2/3 inhibitory activity. Thus, the assays of the present invention may be conducted in the presence or absence of a candidate compound to determine if the candidate compound affects NS2/3 autocleavage. A decrease of detectable NS2 product or NS3 product in the presence of a candidate compound is indicative of NS2/3 inhibition.

Similarly, in another aspect of the present invention there is provided an assay for screening a candidate compound for NS2/3 cleavage inhibitory activity in a sample containing NS2/3 protease, the assay comprising:

a) subjecting a first sample comprising NS2/3 protease to suitable conditions under which at least a portion of NS2/3 is cleaved to yield cleaved NS2 and NS3 products, in the absence of candidate compound;
a') diluting the protease in said first sample to achieve conditions suitable to stop auto-cleavage;
a") immobilizing the NS3 product from step a');
b) subjecting a second sample comprising NS2/3 protease in the presence of a candidate compound under the same conditions as those in step a);
b') diluting the protease in said second sample to achieve conditions suitable to stop auto-cleavage;
b") immobilizing the NS3 product, if any, from step b');
c) incubating immobilized NS3 from step a") with a ligand directly or indirectly labeled with a detectable marker, wherein said ligand preferentially binds to NS3 over NS2/3;
c') incubating immobilized NS3 from step b") with a ligand directly or indirectly labeled with a detectable marker, wherein said ligand binds preferentially to NS3 over NS2/3;
d) determining the amount of immobilized labeled-ligand produced in each of step c) and c'), whereby a decrease in the amount of immobilized labeled-ligand in the step c') as compared to that for step c) indicates that the candidate compound may be an inhibitor of NS2/3 cleavage activity.

Similarly, in another aspect of the present invention there is provided an assay for screening a candidate compound for NS2/3 cleavage inhibitory activity in a sample containing NS2/3 protease, the assay comprising:

a) subjecting a first sample comprising NS2/3 protease to suitable conditions under which at least a portion of NS2/3 is cleaved to yield cleaved NS2 and NS3 products, in the absence of candidate compound;
a') diluting the protease in said first sample to achieve conditions suitable to stop auto-cleavage;
a") immobilizing the NS2 product from step a');
b) subjecting a second sample comprising NS2/3 protease in the presence of a candidate compound under the same conditions as those in step a);
b') diluting the protease in said second sample to achieve conditions suitable to stop auto-cleavage;
b") immobilizing the NS2 product, if any, from step b');
c) incubating immobilized NS2 from step a") with a ligand directly or indirectly labeled with a detectable marker, wherein said ligand preferentially binds to NS2 over NS2/3;
c') incubating immobilized NS2 from step b") with a ligand directly or indirectly labeled with a detectable marker, wherein said ligand preferentially binds to NS2 over NS2/3;
d) determining the amount of immobilized labeled-ligand produced in each of step c) and c'), whereby a decrease in the amount of immobilized labeled-ligand in the step c') as compared to that for step c) indicates that the candidate compound may be an inhibitor of NS2/3 cleavage activity.

NS2/3 protease and variants

The NS2/3 protease 810-1206 **[SEQ ID NO.1]** as well as functionally equivalent variants can be used in embodiments of the present invention. Particularly, examples of variants in accordance with the present invention, are:

(907-1206; **SEQ ID NO. 2**) or (904-1206; **SEQ ID NO. 3**). Particularly, NS2/3 variant (4K-6H-904-1206-ST-4K) of **SEQ ID NO.4** is used in the assay of the present invention.

NS2/3 autocleavage assay conditions

In a first step of the present method, a sample is subjected to conditions under which NS2/3 is cleaved to yield a NS2 product and a NS3 product. Such conditions, including the use of a detergent as an activation agent, are known in the art and suitable conditions are also exemplified herein.

Activation of the refolded NS2/3 protease requires the use of detergents at concentrations at or above their critical micelle concentration, although some detergents do not promote autocleavage. Also, the effect of the detergent on NS2/3 autocleavage activity is enhanced in the presence of glycerol (**2**). The concentration dependence of the NS2/3 protease autocleavage reaction previously reported (**4**) is confirmed using SDS-PAGE/Western blot analysis. At concentrations greater than 200 nM, no concentration dependence is observed (data not shown). The effect of glycerol, pH and DMSO on autocleavage is also evaluated. Similar cleavage kinetics is observed in a buffer containing 20% or 30% glycerol (data not shown). Finally, autocleavage is optimal at pH 7.5 and DMSO has no effect on activity at concentrations ranging from 0.5-5% (data not shown).

Particularly, the NS2/3 is originally prepared in a solution of LDAO to prevent self-cleavage prior to the start of the assay. Particularly, the concentration of LDAO should be well above critical micelle concentration (CMC) in order to block autocleavage. More particularly, in the present assay conditions, LDAO should be present between 0.5 and 1.5% in the solution, most particularly, at about 1%. The NS2/3 solution is afterwards diluted in a solution lacking LDAO, to achieve lower concentrations in order for autocleavage to proceed.

The autocleavage reaction is therefore induced by decreasing the concentration of LDAO, in the presence of an activation agent, the activation agent being a detergent selected from, but not limited to, the group consisting of: CHAPS, Triton X-100, NP-40 and n-dodecyl-β-D-maltoside (DM). Typically, the detergent acting as activation agent is present above its CMC.

Typically, glycerol is present to enhance autocleavage, particularly from 0% to 50%, more particularly, from 20% to 50%, most particularly at 20%.

The NS2/3 protease auto-cleavage is then stopped by transferring an aliquot of the reaction sample in a second reaction vessel thereby diluting the autocleavage reaction mixture and stopping autocleavage. The transfer contributes to stop autocleavage by 1) decreasing the NS2/3 protease concentration, and 2) diluting the amount of activation agent.

In one particular embodiment, the reaction is stopped with a 5-fold dilution of the autocleavage reaction mixture in a buffer containing 50mM HEPES, pH 7.5, 10% glycerol and 1mM TCEP.

Immobilization

Upon transfer of the cleavage mixture containing NS2 product, NS3 product and any remaining uncleaved NS2/3, the cleavage mixture while being diluted (and auto-cleavage thereby stopped) is also preferably immobilized for the purpose of detection using standard means of immobilization. In one embodiment, the NS2/3 protease precursor is tagged on the NS2 portion so as to facilitate immobilization of the cleaved NS2 product which retains the tag on cleavage (any remaining uncleaved NS2/3 will also be immobilized). In another embodiment, the NS2/3 protease precursor is tagged on the NS3 portion. In this case, the cleavage reaction mixture is exposed to an immobilizing surface to which the tagged NS3 will readily bind. Particularly, the corresponding affinity receptor may be coated on the reaction vessel or immobilizing surface to facilitate multiple washings without disrupting the labeled product to be measured.

Preferable pairs of affinity tag/affinity receptor are selected from: MBP/maltose; GST/glutathione; His/ Ni; Strep tag/ streptavidin or neutravidin.

Particularly, the NS2 domain is tagged on the N-terminus with a hexahistidine tag and the reaction vessel is coated with nickel. Preferably, the NS3 domain is tagged on the C-terminus with a Strep tag and the reaction vessel is coated with streptavidin or neutravidin. Particularly, the reaction takes place in 96 well or 384 well plates which have been previously coated with the appropriate affinity receptor. Alternatively, these plates can be purchased from a commercial source (i.e. Pierce).

Specific Ligand

Once the cleavage mixture (either of NS2 or NS3 cleavage product and any remaining uncleaved NS2/3) is immobilized, it is combined with a suitable amount of a specific ligand that preferentially recognizes cleaved NS2 or NS3 over NS2/3 under conditions suitable to permit preferential binding of the specific ligand. Preferential binding of the ligand to the NS2 product or NS3 product and low binding to the uncleaved NS2/3 protease is preferable for an accurate determination of cleavage.

Particularly, the specific ligand is directed against the NS2 cleavage product. Alternatively, the ligand is directed against the NS3 cleavage product. As would be known in the art, the amount of NS2- or NS3-selective ligand to be used in the assay may be determined based on the total possible amount of cleaved NS2 or NS3 that may result in the reaction.

In a particular aspect, the specific ligand is an antibody. Particularly, the antibody is a polyclonal antibody or a monoclonal antibody. Examples of NS2 preferential ligands include antibodies directed to specific amino acid sequences of NS2. Particularly, the specific amino acid sequence used to raise antibodies has a length that is sufficient to induce an immune response and a sequence that is appropriate to raise antibodies that are selective against the NS2 product i.e. that will have low or no cross-reacfivity with the NS3 product and the uncleaved NS2/3. Of course, as will be recognized by a person of skill in the art, such short peptides may need to be conjugated to a carrier protein in order to induce an immune response (as is presented in Example 2 hereinbelow).

In particular, these peptides are selected from the NS2 portion of the protein and may be found by assessing multiple straddling peptides comprising at least 10 consecutive amino acids. Without intending to be limited, for example, short peptides taken from SEQ ID NO.1 may be conjugated and injected to induce an immune response selective for NS2.

In particular, the peptides comprising the following amino acid sequences may be used in accordance with the invention:

Ser-Phe- Glu-Gly-Gln-Gly-Trp-Arg-Leu-Leu **[SEQ ID NO.6]**
Asn-Phe-Glu-Gly-Gln-Gly-Trp-Arg-Leu-Leu **[SEQ ID NO.8];**
Asp-Asn-Phe-Glu-Gly-Gln-Gly-Trp-Arg-Leu **[SEQ ID NO.9];**
Ala-Asp-Asn-Phe-Glu-Gly-Gln-Gly-Trp-Arg **[SEQ ID NO.10];**
Pro-Ala-Asp-Asn-Phe-Glu-Gly-Gln-Gly-Trp **[SEQ ID NO.11];**
Gly-Pro-Ala-Asp-Asn-Phe-Glu-Gly-Gin-Gly **[SEQ ID NO.12];**
Ser-Ala-Arg-Arg-Gly-Arg-Glu-Ile-Leu-Leu **[SEQ ID NO.13];** etc...

Other peptides useful to raise antibodies according to the invention will be readily determined by persons of skill in the art.

In a alternative aspect, the preferential ligand is an antibody directed towards a specific amino acid sequence of NS3. Particularly, the antibody is a polyclonal antibody or a monoclonal antibody. Examples of NS3 preferential ligands include antibodies directed to specific amino acid sequences of NS3. Particularly, the specific amino acid sequence used to raise antibodies has a length that is sufficient to induce an immune response and a sequence that is appropriate to raise antibodies that are selective against the NS3 product i.e. that will have low or no cross-reactivity with the NS2 product and the uncleaved NS2/3. Of course, as will be recognized by a person of skill in the art, such short peptides may need to be conjugated to a carrier protein in order to induce an immune response (as is presented in Example 2 hereinbelow).

In particular, these peptides are selected from the NS3 portion of the protein and may be found by assessing multiple straddling peptides comprising at least 10 consecutive amino acids. Without intending to be limited, for example, short peptides from SEQ ID NO.1 may be conjugated and injected to induce an immune response selective for NS3.

In particular, the peptides comprising the following amino acid sequences may be used in accordance with the invention:

Ala-Pro-Ile-Thr-Ala-Tyr-Ser-Gln-Gln-Thr **[SEQ ID NO.5];**
Pro-Ile-Thr-Ala-Tyr-Ser-Gln-Gln-Thr-Thr **[SEQ ID NO.14];**
Ile-Thr-Ala-Tyr-Ser-Gln-Gln-Thr-Thr-Arg **[SEQ ID NO.15];**
Thr-Ala-Tyr-Ser-Gln-Gln-Thr-Thr-Arg-Gly **[SEQ ID NO.16];**
Arg-Gly-Leu-Leu-Gly-Cys-Ile-Ile-Thr-Ser **[SEQ ID NO.17];** etc...

Other peptides useful to raise antibodies according to the invention will be readily determined by persons of skill in the art.

In one embodiment, the antibody directed against an N-terminal region of NS3 is used as the NS3 preferential ligand, for example, an antibody which is directed against the peptide, APITAYSQQT **[SEQ ID NO.5],** particularly, the K147 polyclonal antibody.

In particular embodiments, the preferential ligand is either a polyclonal antibody or a monoclonal antibody. Particularly, the polyclonal or monoclonal antibody is coupled to a detectable label to facilitate detection of its target molecule. Particularly, the ligand can be detected directly, if it is directly coupled to a detectable label, or alternatively, the specific antibody can be detected indirectly with the use of a second antibody directed against the specific antibody, this second antibody being coupled to a detectable label. Such second antibody can be polyclonal or monoclonal antibodies and can be selected from: monoclonal antibodies (such as anti-IgG, anti-IgM) or polyclonal antibodies (such as: anti-rabbit, anti-mouse, or anti-goat antibodies, etc.) depending on the nature of the specific ligand used to detect the cleavage product. In a particular embodiment, the specific ligand is a polyclonal antibody from rabbit which is detected with the use of an anti-rabbit antibody labeled with Europium.

In another preferred embodiment, the amount of immobilized ligand produced from each of steps c) and c') can be determined by measuring the signal of the label bound directly or indirectly to the immobilized ligand.

Detection

In a particular embodiment of this invention, the NS2- or NS3-preferential ligand is linked (directly or indirectly) to any conventionally used detectable label in order that cleaved NS2 product or NS3 product may be identified and/or quantified. It follows that conventional methods of detection can then be used to detect/measure the detectable label that is bound to immobilized NS2 or NS3. Embodiments of such detectable labels include, for example, radioactive or colorimetric labels that are well known in the art and available in catalogs such as "Amersham Blotting, Labeling and Detection" catalog, GE Healthcare @ www.Amershambiosciences.com.

In one embodiment, the detectable label is a fluorescent marker. The detection/measurement of this detectable label is carried out by methods well known in the art such as is disclosed in "Invitrogen- Molecular Probes Handbook -*A Guide to Fluorescent Probes and Labeling Technology*, $10^{th}$ ed. 2005".

Alternatively, the detectable label is europium that is bound to an anti-rabbit antibody that recognizes the anti-NS3 rabbit antibody referred to above. The detection/measurement of this detectable label is carried out by methods well known in the art such as commercialized by PerkinElmer Life Sciences (DELFIA® system).

It is to be understood in the present embodiment and in the various other embodiments disclosed and claimed herein that the general conditions, including buffers employed, pH of buffers and solutions employed, temperatures employed and time of reaction would include those that do not inhibit the intended various steps and would be readily determinable by persons skilled in the art.

Embodiments of the invention are described by reference to the following specific examples which are not to be construed as limiting:

## EXAMPLES

### Abbreviations

Ab: antibody;
BSA: Bovine serum albumin;
CHAPS: 3-[(3-chloroamidopropyl)dimethyl-ammonio]-1-propanesulfonate;
DMSO: dimethyl sulfoxide;
DM: n-dodecyl-β-D-maltoside;
DTPA: diethylenetriaminepentaacetic acid;
HEPES: 4-(2-hydroxyethyl)-1-piperazineethanesulfonic acid;
LDAO: lauryldiethylamine oxide;
mcKLH: Mari culture keyhole limpet hemocyanin;
NZW: New Zealand White rabbit;
PBS: phosphate buffered saline;
PBS-T: phosphate buffered saline-Tween;
PNPP: para-nitrophenylphosphate;
PVDF: polyvinylidene difluoride;
TCEP: Tris(2-carboxyethyl)phosphine hydrochloride.

### Materials and methods

Assay Reagents

BSA, glycerol, DTPA, zinc chloride, HEPES and DMSO were purchased from Sigma-Aldrich. The detergents DM and LDAO were from Anathrace Inc. and Fluka respectively. DELFIA® reagents were purchased from PerkinElmer Life Sciences.

TCEP was from Pierce, Tween®20 from Bio-Rad and sodium chloride from EM Science.

NS2/3 protease preparation

The expression, production and purification of the NS2/3 protease was carried out according to the procedure previously reported (**2**). Practically speaking, aliquots of the refolded, inactive NS2/3 protein in refolding buffer (50mM Tris, pH 8.0, 0.5 arginine HCl, 1% LDAO, 5mM TCEP) can be stored frozen at -80˚C, and later thawed and diluted in the presence of an activation agent and optionally glycerol, to induce autocleavage.

Generation of polyclonal antibodies

An important aspect of this invention is the use of antibodies as ligand to preferentially bind NS2 or NS3 over NS2/3 in the presence of a mixture of the two.

In order to obtain an antibody that recognizes cleaved NS2 from NS2/3, a peptide corresponding to the C-terminal last 10 amino acid sequence of NS2 is synthesized. The synthetic peptide (SFEGQGWRLL; **SEQ ID NO.6**) is coupled to a carrier protein and used for immunization.

In order to obtain an antibody that recognizes cleaved NS3 from NS2/3, a peptide corresponding to the N-terminal first 10 amino acid sequence of NS3 is synthesized. The synthetic peptide (APITAYSQQT) **[SEQ ID NO.5]** is coupled to a carrier protein and used for immunization.

**Example 1 - Generation of NS3-specific polyclonal antibodies (K147)**

In order to obtain a polyclonal antibody that recognizes cleaved NS3 from NS2/3, a peptide corresponding to the N-terminal first 10 amino acid sequence of NS3 (APITAYSQQT; **SEQ ID NO.5**) is coupled to keyhole limpet hemocyanin (mcKLH) carrier protein and used to immunize rabbits.

Peptide synthesis and Immunogen preparation

The peptide $H_2N$-APITAYSQQT-COOH is purchased from Neo MPS, Inc. (San Diego, CA). To prepare the immunogen, the peptide is conjugated to Mari culture keyhole limpet hemocyanin (mcKLH) carrier protein using the Imject® Immunogen EDC conjugate kit from Pierce. Essentially, 2 mg of the peptide are solubilized in 0.5 ml of Imject® EDC conjugation buffer. The peptide solution is added to 0.2 ml of the reconstituted mcKLH carrier protein solution. Fifty (50) μl of freshly prepared EDC reagent at 10 mg/ml is added to the conjugation reaction and the reaction is then incubated for 2 hours at room temperature. The conjugate is purified by desalting using the desalting column and purification buffer provided in the Imject® immunogen EDC Kit. The fractions containing the immunogen (determined by $OD_{280}$) are pooled and stored at -20˚C.

Immunization

The peptide-carrier protein conjugate is diluted in PBS to achieve 50 μg/ml and emulsified with an equal volume of complete Freund's adjuvant. Two NZW (New Zealand White) male rabbits are immunized sub-cutaneously (s.c.) with the emulsion (50 μg/rabbit in a volume of 2 ml). The rabbits are boosted with the same dose of peptide conjugate emulsified in incomplete Freund's adjuvant at week 4 and 8. Blood is collected 10-14 days after each booster injection and tested for peptide specific antibodies by ELISA. Pre-immune serum is collected one day before the first immunization to use as control serum for each animal. The sera from each rabbit is analyzed by Western blot and by ELISA using peptide antigen-coated plates.

ELISA Assay

For this assay, microtiter plates (NUNC) are coated with the peptide antigen (100 μl per well of a 12.5 μg/ml solution) overnight at 4˚C. The plates are washed three times with 200 μl blocking buffer containing PBS, 3% BSA and 0.05% Tween-20 and incubated for one hour with 200 μl of blocking buffer at room temperature. The wells are washed three times with PBS containing 0.05% Tween-20 (PBS-T). The wells containing the peptide are then incubated with serial dilutions of the rabbit antiserum (1/5-1/390,625) in PBS-T for 2 hours at room temperature. A dilution curve for the pre-immune serum is also tested in parallel. The wells are washed 3 times with 200 μl PBS-T and then incubated with 100 μl of goat anti-rabbit alkaline phosphatase conjugate (1/5000) (Gibco BRL) for 1 hour at room temperature. The wells are washed twice with PBS-T and rinsed with 200 μl of PNPP buffer. This step is followed by an incubation with 100 μl of 4-nitrophenyl phosphate (5 ng/ml diluted in PNPP buffer). The optical absorbance is read at 405 nm.

**Figure 2A** illustrates the antibody dilution curve obtained from the bleed from one animal following the second boost and shows that the antiserum reacts with the peptide antigen in the ELISA. The titer of the antisera against the peptide (1:625) antigen is maintained between the first and second boost. Both immunized rabbits raise an immune response against the peptide antigen as measured by ELISA.

Western blot

NS2/3 protein samples are submitted to the autocleavage reaction and used to evaluate the ability of the polyclonal antibody to recognize preferentially NS3 cleaved product from the NS2/3 protease by Western blot. Protein samples (100 ng) before (-) and after (+) NS2/3 autocleavage reaction are separated by electrophoreses on 15% polyacrilamide gels. The proteins are transferred onto a PVDF membrane by electroblotting. The membranes are blocked with 5% skim milk in PBS-Tween (phosphate buffer saline with 0.05% Tween-20). The membrane is then incubated with the primary antibody for 1 hour at room temperature. The K147 antiserum is diluted 1:1000 while the control polyclonal antibody to NS3 [K135] (Thibeault *et al.*, 2001) is diluted at 1:5000. After washing 4 times, the blots are incubated with 1:20,000 goat anti-rabbit HRP-conjugated secondary antibody (Gibco BRL) for 1 hour at room temperature. After 4 washes, the reaction is visualized using ECLplus western blotting substrate from Pierce and the chemiluminescence signal read on a STORM® Image analysis system (Amersham).

The signal observed in **Figure 2B** with the samples incubated with the K147 antiserum to the N-terminus of NS3 demonstrates that this antibody can recognize preferentially NS3 when cleaved (+) from NS2/3 protease since no significant signal is observed in lane (-) where the uncleaved NS2/3 is recognized by the control anti-NS3 antibody directed against the catalytic domain (K135; panel C). The selectivity of this antiserum is further demonstrated in the 96 well format for NS2/3 autocleavage assay described in Example 2.

From the two rabbits that produce an immune response, one rabbit produces an antiserum that is selective for NS3.

The immunization protocol is then repeated a second time with 6 rabbits. This time, 4 rabbits produce an antiserum that is selective for NS3.

**Example 2 - Evaluation of polyclonal antibody (K147)**

The assay conditions described below are used to evaluate polyclonal antibodies for their ability to discriminate between NS2/3 protease and the NS3 product.

NS2/3 protease autocleavage

The autocleavage reaction is initiated by adding 20 μL of autocleavage buffer (50 mM HEPES, pH 7.5, 30% glycerol, 0.5% DM, 1 mM TCEP) to 30 μL of NS2/3 protease (**SEQ ID NO.4** diluted to a final concentration of 0.2 μM in 50 mM HEPES, pH 7.5, 30% glycerol, 1 mM TCEP). The reaction mixture is incubated for 90 minutes at 30˚C. In the blank reaction, the DM-containing buffer is added just prior to the transfer step. Alternatively, as a negative control, the active-site mutated NS2/3 [H952A] is used to confirm that the antibody minimally cross-reacts with uncleaved NS2/3.

Antibody evaluation for the detection step with a $Eu^{+3}$-labeled anti-rabbit Ab

In a 96-well neutravidin coated plate (purchased from Pierce), 20 μl of the autocleavage mixture is added to 80 μL of 50 mM HEPES, pH 7.5, 10% glycerol, 1 mM TCEP. The assay mixture is incubated for 60 min at room temperature. The plate is then washed three times with 200 μL PBS, 0.05% Tween-20. Then, 100 μL PBS, 0.05% Tween-20, 3% BSA is added per well followed by a 30-min incubation at room temperature and three washes as described above.

100 μL of the polyclonal antibody diluted 1/500 in PBS, pH 7.5, 0.05% Tween-20, 0.3% BSA is added per well followed by a 30-min incubation at room temperature. The plate is then washed three times as described above.

To detect binding of the polyclonal antibody, 100 μL of DELFIA® Eu-N1 labeled anti-rabbit antibody (PerkinElmer Life Sciences) diluted to 8nM in PBS, pH 7.5, 0.05% Tween-20, 0.3% BSA, 100 μM DTPA is added per well followed by a 30-min incubation at room temperature. The plate is then washed three times with 200 μL of the DELFIA® Wash Buffer (PerkinElmer Life Sciences). Finally, 100 μL of the DELFIA® Enhancement Solution (PerkinElmer Life Sciences) is added to each well followed by an incubation of at least 15 min at room temperature. The time-resolved fluorescence is monitored on a Wallac Victor® 1420 Multilabel HTS Counter (PerkinElmer Life Sciences) equipped with an excitation filter at 340 nm and an emission filter at 615 nm.

Using this assay format, an increase in fluorescence is observed upon NS2/3 autocleavage, while no increase in fluorescence is observed with the NS2/3 protease active-site mutant H952A (**Figure 3**) indicating that K147 is selective for cleaved NS3 and that its detection correlates with NS2/3 autocleavage activity.

**Example 3 - Protocol for NS2/3 protease time-resolved fluorescence assay**

The autocleavage reaction is initiated by adding 10 μL of NS2/3 protease (**SEQ ID NO.4** diluted to a final concentration of 800 nM in 50 mM HEPES, pH 7.5, 20% glycerol, 1 mM TCEP) to 30 μL of 50 mM HEPES, pH 7.5, 20% glycerol, 0.266% n-dodecyl-β-D-maltoside, 1 mM TCEP with the final DMSO content kept at 5%. The reaction mixture is incubated for 45 minutes at room temperature.

In the blank wells, autocleavage is prevented by adding $ZnCl_2$ at 10μM or NS4A peptide [**SEQ ID NO. 7**] at 50μM.

A final NS2/3 protease concentration of 200nM is selected based on the concentration dependence of the autocleavage as shown in **Figure 4**. A time-course of the autocleavage reaction is shown in **Figure 5**.

In a 96-well neutravidin-coated plate (purchased from Pierce), 10 μL of the autocleavage mixture is added to 40 μL of 50 mM HEPES, pH 7.5, 10% glycerol, 1 mM TCEP. The assay mixture is incubated for 60 min at room temperature. The plate is then washed three times with 100 μL of 50 mM HEPES, pH 7.5, 0.15 M NaCl, 0.05% Tween-20. Then, 50 μL of the polyclonal antibody K147 diluted up to 6000-fold in 50 mM HEPES, pH 7.5, 0.15 M NaCl, 0.05% Tween®-20, 0.3% BSA is added per well followed by a 30-min incubation at room temperature. The plate is then washed three times as described above. Then, 50 μL of 1.0 nM DELFIA Eu-N1 labeled anti-rabbit antibody (PerkinElmer Life Sciences) diluted in 50 mM HEPES, pH 7.5, 0.15 M NaCl, 0.05% Tween®-20, 0.3% BSA, 100 μM DTPA is added per well followed by a 30-min incubation at room temperature. The plate is then washed three times with 100 μL of the DELFIA® wash buffer. Finally, 50 μL of the DELFIA® enhancement solution is added to each well followed by an incubation of at least 15 min at room temperature. The time-resolved fluorescence is monitored on a Wallac Victor® 1420 Multilabel HTS Counter (PerkinElmer Life Sciences) equipped with an excitation filter at 340 nm and an emission filter at 615 nm. An increase in fluorescence is observed with increasing concentrations of the antibody K147 and with control-to-blank ratios ranging from 18 to 20, clearly showing the ability of the antibody to discriminate between the NS2/3 precursor and the NS3 product (**Figure 6**). A schematic representation of the assay is shown in **Figure 7A**.

In conclusion, in the NS2/3 protease time-resolved fluorescence assay presented herein, the NS2/3 uncleaved precursor and the NS3 product are both captured on the neutravidin-coated plate via their C-terminal Strep-tag, and the NS3 product is detected by using a rabbit polyclonal antibody able to discriminate between the NS2/3 precursor and the NS3 product and an europium-labeled anti-rabbit antibody.

**Example 4 - Immobilization assay based on NS2 detection**

Likewise to detect the NS2 product, an antibody is raised against the peptide SFEGQGWRLL (**SEQ ID NO. 6**).

The autocleavage reaction is initiated by adding in a 96-well round-bottom polypropylene plate (Falcon) 10 μL of NS2/3 protease (**SEQ ID NO.4** diluted to a final concentration of 800 nM in 50 mM HEPES, pH 7.5, 20% glycerol, 1 mM TCEP) to 30 μL of 50 mM HEPES, pH 7.5, 20% glycerol, 0.266% DM, 1 mM TCEP with the final DMSO content kept at 5%. The reaction mixture is incubated for 45 minutes at room temperature. In the negative control wells, autocleavage is prevented by adding $ZnCl_2$ at 10 μM or NS4A peptide (**SEQ ID NO. 7**) at 50 μM.

In a 96-well nickel-coated plate (purchased from Pierce), 10 μL of the autocleavage mixture is added to 40 μl of 50 mM HEPES, pH 7.5, 10% glycerol, 1 mM TCEP. The assay mixture is incubated for 60 minutes at room temperature. The plate is then washed three times with 100 μL of 50 mM HEPES, pH 7.5, 0.15M NaCl, 0.05% Tween®-20. Then, 50 μL of the anti-NS2 antibody (previously titrated to determine the dilution factor) diluted in 50 mM HEPES, pH 7.5, 0.15M NaCl, 0.05% Tween®-20, 0.3% BSA is added per well followed by a 30-min incubation at room temperature. The plate is then washed three times as described above. Then, 50 μL of 1.0 nM DELFIA® Eu-N1 labeled anti-mouse antibody (PerkinElmer Life Sciences) diluted in 50 mM HEPES, pH 7.5, 0.15M NaCl, 0.05% Tween®-20, 0.3% BSA, 100 μM DTPA is added per well followed by a 30-min incubation at room temperature. The plate is then washed three times with 100 μL of the DELFIA® wash buffer (PerkinElmer Life Sciences). Finally, 50 μL of the DELFIA® enhancement solution (PerkinElmer Life Sciences) is added to each well followed by an incubation of at least 15 minutes at room temperature. The time-resolved fluorescence is monitored on a Wallac Victor® 1420 Multilabel HTS Counter (PerkinElmer Life Sciences) equipped with an excitation filter at 340 nm at an emission filter at 615 nm. A schematic representation of the assay is shown in **Figure 7B.**

**Example 5 - Protocol for ultra High Throughput Screening**

The autocleavage reaction is initiated by adding in a 384-well round-bottom polypropylene plate (Greiner): 10 μl of NS2/3 protease (**SEQ ID NO.4** diluted to a final concentration of 600 nM in 50 mM HEPES, pH 7.5, 20% glycerol, 1 mM TCEP) to 10 μL of the test compound in DMSO (diluted in 50 mM HEPES, pH 7.5, 20% glycerol, 1 mM TCEP) and 10 μL of 50 mM HEPES, pH 7.5, 20% glycerol, 0.6% DM, 1 mM TCEP. The final DMSO content is kept at 5%. The reaction mixture is incubated for 45 minutes at room temperature. In the blank wells, autocleavage is prevented by adding $ZnCl_2$ at 10 μM or NS4A peptide (**SEQ ID NO. 7**) at 50 μM.

In a 384-well neutravidin-coated plate (purchased from Pierce), 5 μL of the autocleavage mixture is added to 20 μL of 50 mM HEPES, pH 7.5, 10% glycerol, 1 mM TCEP. The assay mixture is incubated for 60 minutes at room temperature. The plate is then washed three times with 50 μL of 50 mM HEPES, pH 7.5, 0.15M NaCl, 0.05% Tween®-

20. Then, 25 μL of the polyclonal antibody K147 diluted to 0.03 μg./ml in 50 mM HEPES, pH 7.5, 0.15M NaCl, 0.05% Tween®-20, 0.3% BSA is added per well followed by a 30-min incubation at room temperature. The plate is then washed three times as described above. Then, 25 μL of DELFIA® Eu-N1 labeled anti-rabbit antibody (PerkinElmer Life Sciences) diluted to 1.0 nM in 50 mM HEPES, pH 7.5, 0.15M NaCl, 0.05% Tween®-20, 0.3% BSA, 100 μM DTPA is added per well followed by a 30-min incubation at room temperature. The plate is then washed three times with 50 μL of the DELFIA® wash buffer (PerkinElmer Life Sciences).

Alternatively, 25 μL of the antibodies solution composed of: 1) 0.03 μg/mL of the K147 polyclonal antibody previously purified on a peptide column using APITAYSQQT as ligand, and 2) 0.5 nM DELFIA® Eu-N1 labeled anti-rabbit antibody (PerkinElmer Life Sciences) diluted in 50 mM HEPES, pH 7.5, 0.15M NaCl, 0.05% Tween®-20, 0.3% BSA, 100 μM DTPA is added per well followed by a 30-min incubation at room temperature. The plate is then washed three times with 50 μL of the DELFIA® wash buffer (PerkinElmer Life Sciences). Finally, 25 μL of the DELFIA® enhancement solution (PerkinElmer Life Sciences) is added to each well followed by an incubation of at least 15 minutes at room temperature. The time-resolved fluorescence is monitored on a Wallac Victor® 1420 Multilabel HTS Counter (PerkinElmer Life Sciences) equipped with an excitation filter at 340 nm at an emission filter at 615 nm. Assay statistics are presented in **Figure 8** where it can be seen that a signal window of 20-25 is observed between the positive control (maximal NS2/3 protease activity) and the blank (background NS2/3 protease activity) with a Z' of 0.70-0.75 [Z' being a statistical parameter defined in (**7**)]. **Figure 9** shows the results obtained with the same assay on test compound **A** diluted at different concentrations. The level of inhibition (% inhibition) of each well containing inhibitor was calculated with the following equation:

$$\%\ inhibition = \left(1 - \left[\frac{cps_{well} - cps_{blank}}{cps_{control} - cps_{blank}}\right]\right) * 100$$

The percentage of inhibition was plotted against compound **A** concentration and a nonlinear curve was fitted to the percent inhibition-concentration data according to the Hill model. The calculated percent inhibition values were then used to determine the median effective concentration $IC_{50}$, the slope factor (n) and the maximum inhibition ($I_{max}$) by the NLIN procedure of the SAS software (Statistical Software System; SAS Institute, Inc., Cary, N.C.) using the following equation:

$$\%\ inhibition = \frac{I_{max} \times [inhibitor]^n}{[inhibitor]^n + IC_{50}{}^n}$$

An $IC_{50}$ of approximately 31 μM is obtained for compound **A**.

## References

(**1**) Kolykhalov, A.A., Mihalik, K., Feinstone, S.M. and C.M. Rice. (2000) Hepatitis C virus-encoded enzymatic activities and conserved RNA elements in the 3'-nontranslated region are essential for virus replication in vivo. J. Virol. 74: 2046-2051.

(**2**) Thibeault, D., Maurice, R., Pilote, L., Lamarre, D. and Pause, A. (2001) In vitro characterization of a purified NS2/3 protease variant of hepatitis C virus. J. Biol. Chem. 276 (49): 46678-46684.

(**3**) Boehringer Ingelheim (Canada) Ltd. US Patent 6,815,159 (9 November 2004) Purified active HCV NS2/3 protease (corresponding to WO 02/48375).

(**4**) Pallaoro, M., Lahm, A., Biasiol, G., Brunetti, M., Nardella, C., Orsatti, L., Bonelli, F., Orrù, S, Narjes, F. and Steinkühler, C. (2001) Characterization of the hepatitis C virus NS2/3 processing reaction by using a purified precursor protein. J. Virol. 75: 9939-9946.

(**5**) Istituto di ricerche di biologia molecolare P. Angeletti, Italy. Patent application WO 01/68818 A2 (priority 17 March 2000), HCV NS2/3 fragments and uses thereof.

(**6**) Waugh, D.S. 2005; Making the most of affinity tags; Trends Biotechnology 23: 316-320.

(**7**) Zhang J.-H., Chung, T.D.Y., Oldenburg K.R. (1999) A Simple Statistical Parameter for Use in Evaluation and Validation of High Throughput Screening, J. of Biomol. Screening 4(2): 67-73.

## Claims

1. A method for detecting a NS2/3 autocleavage product in a sample containing NS2/3 protease, the method comprising:

    a) subjecting the sample to suitable conditions under which at least a portion of the NS2/3 protease is self-cleaved to yield cleaved NS2 and NS3 products;
    b) incubating the NS2 and NS3 products with either of:

        i) a NS2-selective antibody directed against a peptide consisting of SEQ ID NO: 6 under conditions suitable to afford binding of the NS2-selective antibody to said cleaved NS2 product to form NS2-bound antibody; or
        ii) a NS3-selective antibody directed against a peptide consisting of SEQ ID NO: 5 under conditions suitable to afford binding of the NS3-selective antibody to the cleaved NS3 product to form NS3-bound antibody; and

    c) detecting the amount of NS2-bound antibody or NS3-bound antibody produced in step b), whereby the amount detected correlates with the NS2/3 autocleavage activity.

2. The method according to claim 1, wherein step b) involves Incubating the NS2 and NS3 products with a NS2-selective antibody directed against a peptide consisting of SEQ ID NO: 6 under conditions suitable to afford binding of the NS2-selective antibody to said cleaved NS2 product to form NS2-bound antibody.

3. The method according to claim 1, wherein step b) involves incubating the NS2 and NS3 products with a NS3-selective antibody directed against a peptide consisting of SEQ ID NO: 5 under conditions suitable to afford binding of the NS3-selective antibody to said cleaved NS3 product to form NS3-bound antibody.

4. The method according to claim 1,2 or 3, wherein lauryldiethylamine oxide (LDAO) is initially present in said NS2/3 sample to prevent autocleavage.

5. The method according to claim 4, wherein said LDAO is present at a concentration of at least 1% $\pm$ 10%.

6. The method according to claims 4 or 5, wherein in step a) said sample is diluted such that LDAO concentration is decreased at least 1.5 fold $\pm$ 10% and is incubated in the presence of an activation agent to induce autocleavage.

7. The method according to claim 6, wherein said activation agent is a detergent selected from the group consisting of: CHAPS, Triton X-100, NP-40 and n-dodecyl-β-D-maltoside (DM).

8. The method according to claim 7, wherein said detergent acting as activation agent is present above its critical micelle concentration (CMC).

9. The method according to any one of claims 1 to 8, wherein in step a), said protease is incubated in a solution further comprising glycerol to enhance autocleavage.

10. The method according to claim 9, wherein said glycerol is present at a concentration of 20% $\pm$ 10%.

11. The method according to any one of claims 1 to 10, wherein said antibody is a polyclonal antibody or a monoclonal antibody.

12. The method according to any one of claims 1 to 11, wherein said antibody is detected with the use of a detectable label.

13. The method according to claim 12, wherein said detectable label is selected from the group consisting of: fluorescent label, chemiluminescent label, colorimetric label, enzymatic marker, and radioactive isotope.

14. The method according to claim 13, wherein said detectable label is europium.

15. The method according to claim 12, 13 or 14, wherein said detectable label is linked directly to said antibody.

16. A method for detecting NS3 product in a sample containing NS2/3 protease in the presence of 1% ± 10% lauryldiethylamine oxide (LDAO), comprising:

a) diluting said sample in the presence of an activation agent to allow at least a portion of the NS2/3 protease to self-cleave to yield cleaved NS2 and NS3 products;
a') further diluting said sample to stop auto-cleavage;
a") immobilizing the NS3 product from step a');
b) incubating the immobilized NS3 product of step a") with a NS3-selective antibody directed against a peptide consisting of SEQ ID NO: 5:
c) detecting the immobilized NS3-bound antibody produced in step b), whereby the amount of antibody detected correlates with the NS2/3 autocleavage activity.

17. The method according to claim 16, wherein step a') and a") can be performed in sequence, or in inverse sequence, or together in the same step, and wherein said dilution of said sample in step a') can be carried out by washing said immobilized sample.

18. The method of claim 16 or 17, wherein said antibody is labeled with europium.

19. An assay for screening a candidate compound for NS2/3 cleavage inhibitory activity in a sample containing NS2/3 protease, the assay comprising:

a) subjecting a first sample comprising NS2/3 protease to suitable conditions under which at least a portion of NS2/3 is self-cleaved to yield cleaved NS2 and NS3 products in the absence of a candidate compound;
b) subjecting a second sample comprising NS2/3 protease in the presence of a candidate compound under the same conditions as those in step a);
c) incubating the first and second samples with either of:

i) an NS2-selective antibody directed against a peptide consisting of SEQ ID NO: 6; or
ii) an NS3-selective antibody directed against a peptide consisting of SEQ ID NO: 5;

under conditions suitable to cause binding of the antibody to NS2 or NS3; and
d) determining the amount of antibody bound to the second sample and to the first sample produced in step c), whereby a decrease in the amount of antibody in the second sample compared to that of the first sample indicates that the candidate compound may be an inhibitor of NS2/3 autocleavage activity.

20. The assay according to claim 19, wherein lauryldiethylamine oxide (LDAO) is initially present in said NS2/3 sample to prevent autocleavage.

21. The assay according to claim 20, wherein said LDAO is present at a concentration of 1% ± 10%.

22. The assay according to claim 20 or 21, wherein in step a) said sample is diluted such that LDAO concentration is decreased at least 1.5 fold 10% and is incubated in the presence of an activation agent to induce autocleavage.

23. The assay according to claim 22, wherein said activation agent is a detergent selected from the group consisting of: CHAPS, Triton X-100, NP-40 and n-dodecyl-β-D-maltoside (DM).

24. The assay according to claim 23, wherein said detergent acting as activation agent is present above its critical micelle concentration (CMC).

**25.** The assay according to any one of claims 19 to 24, wherein in step a), said protease is incubated in a solution further comprising glycerol to enhance autocleavage.

**26.** The assay according to claim 25, wherein said glycerol is present at a concentration of 20% ± 10%.

**27.** The assay according to any one of claims 19 to 26, wherein said antibody is a polyclonal antibody or a monoclonal antibody.

**28.** The assay according to any one of claims 19 to 27, wherein said antibody is detected with the use of a detectable label.

**29.** The assay according to claim 28, wherein said detectable label is selected from the group consisting of: fluorescent label, chemiluminescent label, colorimetric label, enzymatic marker, and radioactive isotope.

**30.** The assay according to claim 29, wherein said detectable label is europium.

**31.** The assay according to claim 28, 29 or 30, wherein said detectable label is linked directly to said antibody.

**32.** An assay for screening a candidate compound for NS2/3 cleavage inhibitory activity in a sample containing NS2/3 protease in the presence of 1% ± 10% lauryldiethylamine oxide (LDAO), the assay comprising:

a) diluting a first sample comprising NS2/3 protease in the presence of an activation agent to allow at least a portion of NS2/3 is cleaved to yield cleaved NS2 and NS3 products, in the absence of candidate compound;
a') further diluting the protease in said first sample to stop auto-cleavage;
a") immobilizing the NS3 product from step a');
b) subjecting a second sample comprising NS2/3 protease in the presence of a candidate compound under the same conditions as those in step a);
b') diluting the protease in said second sample to achieve the same conditions as those in step a');
b") immobilizing the NS3 product, if any, from step b');
c) incubating immobilized NS3 from step a") with an antibody directed against the peptide APITAYSQQT [**SEQ ID NO.5**], said antibody being directly or indirectly labeled with a detectable marker;
c') incubating immobilized NS3 from step b") with the same antibody as in step c);
d) determining the amount of labeled-antibody immobilized in each of step c) and c'), whereby a decrease in the amount of immobilized labeled-antibody in the step c') as compared to that for step c) indicates that the candidate compound may be an inhibitor of NS2/3 cleavage activity.

**33.** The assay according to claim 32, wherein step a') and a") can be performed in sequence, or in inverse sequence, or together in the same step, and wherein said dilution of said sample in step a') can be carried out by washing said immobilized sample.

**34.** The assay of claim32 or 33, wherein said antibody is labeled with europium.

**35.** An antibody directed against a peptide of SEQ ID NO: 5, said antibody selective for NS3 cleaved product and having no or low cross-reactivity with NS2 cleaved product and uncleaved NS2/3 as determined by a signal that is at least 2 fold higher ± 10% against NS3 cleaved product than against NS2 product and uncleaved NS2/3.

**36.** An antibody directed against a peptide of SEQ **ID** NO: 6, said antibody selective for NS2 cleaved product and having no or low cross-reactivity with NS3 cleaved product and uncleaved NS2/3 as determined by a signal that is at least, 2 fold higher ± 10% against NS2 cleaved product than against NS3 product and uncleaved NS2/3.

## Patentansprüche

**1.** Verfahren zum Nachweisen eines NS2/3-Selbstspaltungsprodukts in einer Probe, die eine NS2/3-Protease enthält, wobei das Verfahren folgendes umfasst:

a) die Probe wird geeigneten Bedingungen unterworfen, bei denen mindestens ein Teil der NS2/3-Protease der Selbstspaltung unter Bildung von gespaltenen NS2- und NS3-Produkten unterliegt;
b) die NS2- und NS3-Produkte werden inkubiert mit entweder

i) einem NS2-selektiven Antikörper, der gegen ein Peptid, das aus SEQ ID NO: 6 besteht, gerichtet ist, unter Bedingungen, die dazu geeignet sind, die Bindung des NS2-selektiven Antikörpers an das gespaltene NS2-Produkt unter Bildung von NS2-gebundenem Antikörper herbeizuführen; oder
ii) einem NS3-selektiven Antikörper, der gegen ein Peptid gerichtet ist, das aus SEQ ID NO: 5 besteht, unter Bedingungen, die dazu geeignet sind, die Bindung des NS3-selektiven Antikörpers an das gespaltene NS3-Produkt unter Bildung von NS3-gebundenem Antikörper herbeizuführen; und

c) die Menge von NS2-gebundenem Antikörper oder NS3-gebundenem Antikörper, die in Stufe b) gebildet worden ist, wird nachgewiesen, wobei die nachgewiesene Menge mit der NS2/3-Selbstspaltungsaktivität korreliert.

2. Verfahren nach Anspruch 1, wobei die Stufe b) das Inkubieren der NS2- und NS3-Produkte mit einem NS2-selektiven Antikörper gegen ein Peptid, das aus SEQ ID NO: 6 besteht, unter Bedingungen beinhaltet, die dazu geeignet sind, die Bindung des NS2-selektiven Antikörpers an das gespaltene NS2-Produkt unter Bildung von NS2-gebundenem Antikörper herbeizuführen.

3. Verfahren nach Anspruch 1, wobei die Stufe b) das Inkubieren der NS2- und NS3-Produkte mit einem NS3-selektiven Antikörper gegen ein Peptid, das aus SEQ ID NO: 5 besteht, unter Bedingungen beinhaltet, die dazu geeignet sind, die Bindung des NS3-selektiven Antikörpers an das gespaltene NS3-Produkt unter Bildung von NS3-gebundenem Antikörper herbeizuführen.

4. Verfahren nach Anspruch 1, 2 oder 3, wobei Lauryldiethylaminoxid (LDAO) zu Beginn in der NS2/3-Probe vorhanden ist, um die Selbstspaltung zu verhindern.

5. Verfahren nach Anspruch 4, wobei das LDAO in einer Konzentration von mindestens 1 % $\pm$ 10 % vorhanden ist.

6. Verfahren nach Anspruch 4 oder 5, wobei in Stufe a) die Probe so verdünnt wird, dass die LDAO-Konzentration mindestens 1,5-fach $\pm$ 10% vermindert wird, und in Gegenwart eines Aktivierungsmittels inkubiert wird, um die Selbstspaltung zu induzieren.

7. Verfahren nach Anspruch 6, wobei es sich beim Aktivierungsmittel um ein Detergens handelt, das aus der Gruppe ausgewählt wird, die besteht aus: CHAPS, Triton X-100, NP-40 und n-Dodecyl-β-D-maltosid (DM).

8. Verfahren nach Anspruch 7, wobei das Detergens, das als Aktivierungsmittel wirkt, in einer Menge vorhanden ist, die über einer kritischen Mizellenkonzentration (CMC) liegt.

9. Verfahren nach einem der Ansprüche 1 bis 8, wobei in Stufe a) die Protease in einer Lösung inkubiert wird, die ferner Glycerin enthält, um die Selbstspaltung zu verstärken.

10. Verfahren nach Anspruch 9, wobei das Glycerin in einer Konzentration von 20 % $\pm$ 10 % vorhanden ist.

11. Verfahren nach einem der Ansprüche 1 bis 10, wobei es sich beim Antikörper um einen polyklonalen Antikörper oder um einen monoklonalen Antikörper handelt.

12. Verfahren nach einem der Ansprüche 1 bis 11, wobei der Antikörper unter Verwendung einer nachweisbaren Markierung nachgewiesen wird.

13. Verfahren nach Anspruch 12, wobei die nachweisbare Markierung aus der Gruppe ausgewählt wird, die besteht aus: einer fluoreszierenden Markierung, einer chemilumineszierenden Markierung, einer kolorimetrischen Markierung, einem Enzymmarker oder einem radioaktiven Isotop.

14. Verfahren nach Anspruch 13, wobei es sich bei der nachweisbaren Markierung um Europium handelt.

15. Verfahren nach Anspruch 12, 13 oder 14, wobei die nachweisbare Markierung direkt mit dem Antikörper verknüpft ist.

16. Verfahren zum Nachweisen von NS3-Produkt in einer Probe, die NS2/3-Protease enthält, in Gegenwart von 1 % $\pm$ 10 % Lauryldiethylaminoxid (LDAO), umfassend:

a) das Verdünnen der Probe in Gegenwart eines Aktivierungsmittels, um die Selbstspaltung mindestens eines Teils der NS2/3-Protease unter Bildung von gespaltenen NS2- und NS3-Produkten zuzulassen;
a') das weitere Verdünnen der Probe zum Stoppen der Selbstspaltung;
a") das Immobilisieren des NS3-Produkts von Stufe a');
b) das Inkubieren des immobilisierten NS3-Produkts von Stufe a") mit einem NS3-selektiven Antikörper gegen ein Peptid, das aus SEQ ID NO: 5 besteht;
c) das Nachweisen des immobilisierten, NS3-gebundenen Antikörpers, der in Stufe b) gebildet worden ist, wobei die Menge des nachgewiesenen Antikörpers mit der NS2/3-Selbstspaltungsaktivität korreliert.

**17.** Verfahren nach Anspruch 16, wobei die Stufen a') und a") nacheinander oder in umgekehrter Reihenfolge oder zusammen in der gleichen Stufe durchgeführt werden können und wobei die Verdünnung der Probe in Stufe a') durch Waschen der immobilisierten Probe durchgeführt werden kann.

**18.** Verfahren nach Anspruch 16 oder 17, wobei der Antikörper mit Europium markiert wird.

**19.** Test zum Screening einer als Kandidat in Frage kommenden Verbindung auf Hemmaktivität der NS2/3-Spaltung in einer Probe mit einem Gehalt an NS2/3-Protease, wobei der Test folgendes umfasst:

a) eine erste Probe, die NS2/3-Protease enthält, wird geeigneten Bedingungen unterworfen, bei denen mindestens ein Teil des NS2/3 der Selbstspaltung unter Bildung von gespaltenen NS2- und NS3-Produkten in Abwesenheit einer als Kandidat in Frage kommenden Verbindung unterliegt;
b) eine zweite Probe, die NS2/3-Protease umfasst, wird in Gegenwart einer als Kandidat in Frage kommenden Verbindung den gleichen Bedingungen wie in Stufe a) unterworfen;
c) die erste und die zweite Probe werden mit entweder

i) einem NS2-selektiven Antikörper gegen ein Peptid, das aus SEQ ID NO: 6 besteht; oder
ii) einem NS3-selektiven Antikörper gegen ein Peptid, das aus SEQ ID NO: 5 besteht;

unter Bedingungen inkubiert, die dazu geeignet sind, die Bindung des Antikörpers an NS2 oder NS3 zu verursachen; und
d) die Menge des in Stufe c) erzeugten Antikörpers, der an die zweite Probe und an die erste Probe gebunden ist, wird bestimmt, wobei eine Abnahme der Menge des Antikörpers in der zweiten Probe im Vergleich mit der Menge der ersten Probe anzeigt, dass es sich bei der als Kandidat in Frage kommenden Verbindung um einen Inhibitor der NS2/3-Selbstspaltungsaktivität handeln kann.

**20.** Test nach Anspruch 19, wobei Lauryldiethylaminoxid (LDAO) zu Beginn in der NS2/3-Probe vorhanden ist, um die Selbstspaltung zu verhindern.

**21.** Test nach Anspruch 20, wobei das LDAO in einer Konzentration von 1 % $\pm$ 10 % vorhanden ist.

**22.** Test nach Anspruch 20 oder 21, wobei in Stufe a) die Probe so verdünnt wird, dass die LDAO-Konzentration mindestens 1,5-fach $\pm$ 10% vermindert wird, und in Gegenwart eines Aktivierungsmittels inkubiert wird, um die Selbstspaltung zu induzieren.

**23.** Test nach Anspruch 22, wobei es sich beim Aktivierungsmittel um ein Detergens handelt, das aus der Gruppe ausgewählt wird, die besteht aus: CHAPS, Triton X-100, NP-40 und n-Dodecyl-β-D-maltosid (DM).

**24.** Test nach Anspruch 23, wobei das Detergens, das als Aktivierungsmittel wirkt, in einer Menge vorhanden ist, die über einer kritischen Mizellenkonzentration (CMC) liegt.

**25.** Test nach einem der Ansprüche 19 bis 24, wobei in Stufe a) die Protease in einer Lösung inkubiert wird, die ferner Glycerin enthält, um die Selbstspaltung zu verstärken.

**26.** Test nach Anspruch 25, wobei das Glycerin in einer Konzentration von 20 % $\pm$ 10 % vorhanden ist.

**27.** Test nach einem der Ansprüche 19 bis 26, wobei es sich beim Antikörper um einen polyklonalen Antikörper oder um einen monoklonalen Antikörper handelt.

**28.** Test nach einem der Ansprüche 19 bis 27, wobei der Antikörper unter Verwendung einer nachweisbaren Markierung nachgewiesen wird.

**29.** Test nach Anspruch 28, wobei die nachweisbare Markierung aus der Gruppe ausgewählt wird, die besteht aus: einer fluoreszierenden Markierung, einer chemilumineszierenden Markierung, einer kolorimetrischen Markierung, einem Enzymmarker oder einem radioaktiven Isotop.

**30.** Test nach Anspruch 29, wobei es sich bei der nachweisbaren Markierung um Europium handelt.

**31.** Test nach Anspruch 28, 29 oder 30, wobei die nachweisbare Markierung direkt mit dem Antikörper verknüpft ist.

**32.** Test zum Screening einer als Kandidat in Frage kommenden Verbindung auf Hemmaktivität der NS2/3-Spaltung in einer Probe, die NS2/3-Protease enthält, in Gegenwart von 1 % ± 10 % Lauryldiethylaminoxid (LDAO), wobei der Test folgendes umfasst:

a) eine erste Probe, die NS2/3-Protease umfasst, wird in Gegenwart eines Aktivierungsmittels verdünnt, um die Spaltung mindestens eines Teils von NS2/3 unter Bildung von gespaltenen NS2- und NS3-Produkten in Abwesenheit einer als Kandidat in Frage kommenden Verbindung zu ermöglichen;
a') die Protease in der ersten Probe wird weiter verdünnt, um die Selbstspaltung zu stoppen;
a") das NS3-Produkt von Stufe a') wird immobilisiert;
b) eine zweite Probe, die NS2/3-Protease umfasst, wird in Gegenwart einer als Kandidat in Frage kommenden Verbindung den gleichen Bedingungen wie in Stufe a) unterworfen;
b') die Protease in der zweiten Probe wird verdünnt, um die gleichen Bedingungen wie in Stufe a) zu erzielen;
b") das etwaige NS3-Produkt von Stufe b') wird immobilisiert;
c) das immobilisierte NS3 von Stufe a") wird mit einem Antikörper gegen das Peptid APITAYSQQT (SEQ ID NO: 5) inkubiert, wobei der Antikörper direkt oder indirekt mit einem nachweisbaren Marker markiert ist;
c') das immobilisierte NS3 von Stufe b") wird mit dem gleichen Antikörper wie in Stufe c) inkubiert;
d) die Menge des in den einzelnen Stufen c) und c') immobilisierten markierten Antikörpers wird bestimmt, wobei eine Abnahme der Menge des immobilisierten, markierten Antikörpers in Stufe c') im Vergleich zu der Menge von Stufe c) anzeigt, dass es sich bei der als Kandidat in Frage kommenden Verbindung um einen Inhibitor der NS2/3-Spaltungsaktivität handeln kann.

**33.** Test nach Anspruch 32, wobei die Stufen a') und a") nacheinander oder in umgekehrter Reihenfolge oder zusammen in der gleichen Stufe durchgeführt werden können und wobei die Verdünnung der Probe in Stufe a') durch Waschen der immobilisierten Probe durchgeführt werden kann.

**34.** Test nach Anspruch 32 oder 33, wobei der Antikörper mit Europium markiert ist.

**35.** Antikörper gegen ein Peptid von SEQ ID NO: 5, wobei der Antikörper selektiv für NS3-gespaltenes Produkt ist und keine oder eine geringe Kreuzreaktivität mit NS2-gespaltenem Produkt und ungespaltenem NS2/3 zeigt, bestimmt durch ein Signal, das mindestens 2-fach höher ± 10 % gegenüber NS3-gespaltenem Produkt als gegenüber NS2-Produkt und ungespaltenem NS2/3 ist.

**36.** Antikörper gegen ein Peptid von SEQ ID NO: 6, wobei der Antikörper selektiv für NS2-gespaltenes Produkt ist und keine oder eine geringe Kreuzreaktivität mit NS3-gespaltenem Produkt und ungespaltenem NS2/3 aufweist, bestimmt durch ein Signal, das mindestens 2-fach höher ± 10 % gegenüber NS2-gespaltenem Produkt als gegenüber NS3-Produkt und ungespaltenem NS2/3 ist.

## Revendications

**1.** Procédé pour détecter un produit d'autoclivage de NS2/3 dans un échantillon contenant une protéase NS2/3, le procédé comprenant :

a) soumettre l'échantillon à des conditions appropriées dans lesquelles au moins une partie de la protéase NS2/3 est autoclivée pour produire des produits NS2 et NS3 clivés ;
b) incuber les produits NS2 et NS3 avec :

i) un anticorps sélectif pour NS2 dirigé contre un peptide consistant en SEQ ID NO : 6 dans des conditions appropriées pour réaliser la liaison de l'anticorps sélectif pour NS2 audit produit NS2 clivé pour former un anticorps lié à NS2 ; ou

ii) un anticorps sélectif pour NS3 dirigé contre un peptide consistant en SEQ ID NO : 5 dans des conditions appropriées pour réaliser la liaison de l'anticorps sélectif pour NS3 audit produit NS3 clivé pour former un anticorps lié à NS3 ; et

c) détecter la quantité d'anticorps lié à NS2 ou d'anticorps lié à NS3 produite dans l'étape b), où la quantité détectée est corrélée à l'activité d'autoclivage de NS2/3.

**2.** Procédé selon la revendication 1 où l'étape b) comprend l'incubation des produits NS2 et NS3 avec un anticorps sélectif pour NS2 dirigé contre un peptide consistant en SEQ ID NO : 6 dans des conditions appropriées pour réaliser la liaison de l'anticorps sélectif pour NS2 audit produit NS2 clivé pour former un anticorps lié à NS2.

**3.** Procédé selon la revendication 1 où l'étape b) comprend l'incubation des produits NS2 et NS3 avec un anticorps sélectif pour NS3 dirigé contre un peptide consistant en SEQ ID NO : 5 dans des conditions appropriées pour réaliser la liaison de l'anticorps sélectif pour NS3 audit produit NS3 clivé pour former un anticorps lié à NS3.

**4.** Procédé selon la revendication 1, 2 ou 3 où de l'oxyde de lauryldiéthylamine (LDAO) est initialement présent dans ledit échantillon de NS2/3 pour empêcher l'autoclivage.

**5.** Procédé selon la revendication 4 où ledit LDAO est présent à une concentration d'au moins 1 % $\pm$ 10 %.

**6.** Procédé selon les revendications 4 ou 5 où, dans l'étape a), ledit échantillon est dilué de sorte que la concentration de LDAO est diminuée d'au moins 1,5 fois $\pm$ 10 % et est incubé en présence d'un agent d'activation pour induire l'autoclivage.

**7.** Procédé selon la revendication 6 où ledit agent d'activation est un détergent choisi dans le groupe consistant en : CHAPS, Triton X-100, NP-40 et le n-dodécyl-β-D-maltoside (DM).

**8.** Procédé selon la revendication 7 où ledit détergent jouant le rôle d'agent d'activation est présent au-delà de sa concentration micellaire critique (CMC).

**9.** Procédé selon l'une quelconque des revendications 1 à 8 où, dans l'étape a), ladite protéase est incubée dans une solution comprenant en outre du glycérol pour augmenter l'autoclivage.

**10.** Procédé selon la revendication 9 où ledit glycérol est présent à une concentration de 20 % $\pm$ 10 %.

**11.** Procédé selon l'une quelconque des revendications 1 à 10 où ledit anticorps est un anticorps polyclonal ou un anticorps monoclonal.

**12.** Procédé selon l'une quelconque des revendications 1 à 11 où ledit anticorps est détecté au moyen d'un marqueur détectable.

**13.** Procédé selon la revendication 12 où ledit marqueur détectable est choisi dans le groupe consistant en : un marqueur fluorescent, un marqueur chimiluminescent, un marqueur colorimétrique, un marqueur enzymatique et un isotope radioactif.

**14.** Procédé selon la revendication 13 où ledit marqueur détectable est l'europium.

**15.** Procédé selon la revendication 12, 13 ou 14 où ledit marqueur détectable est lié directement audit anticorps.

**16.** Procédé pour détecter un produit NS3 dans un échantillon contenant une protéase NS2/3 en présence de 1 % $\pm$ 10 % d'oxyde de lauryldiéthylamine (LDAO), comprenant :

a) diluer ledit échantillon en présence d'un agent d'activation pour permettre à au moins une partie de la protéase NS2/3 de s'autocliver pour produire des produits NS2 et NS3 clivés ;

a') diluer encore ledit échantillon pour stopper l'autoclivage ;

a") immobiliser le produit NS3 provenant de l'étape a') ;
b) incuber le produit NS3 immobilisé de l'étape a") avec un anticorps sélectif pour NS3 dirigé contre un peptide consistant en SEQ ID NO : 5 ;
c) détecter l'anticorps lié à NS3 immobilisé produit dans l'étape b),

où la quantité d'anticorps détectée est corrélée à l'activité d'autoclivage de NS2/3.

**17.** Procédé selon la revendication 16 où les étapes a') et a") peuvent être accomplies en succession, ou en succession inversée, ou ensemble dans la même étape, et où ladite dilution dudit échantillon dans l'étape a') peut être conduite par lavage dudit échantillon immobilisé.

**18.** Procédé selon la revendication 16 ou 17 où ledit anticorps est marqué avec l'europium.

**19.** Analyse pour sélectionner un composé candidat pour l'activité inhibant le clivage de NS2/3 dans un échantillon contenant une protéase NS2/3, l'analyse comprenant :

a) soumettre un premier échantillon comprenant une protéase NS2/3 à des conditions appropriées dans lesquelles au moins une partie de NS2/3 est autoclivée pour produire des produits NS2 et NS3 clivés en l'absence d'un composé candidat ;
b) soumettre un second échantillon comprenant une protéase NS2/3 en présence d'un composé candidat aux mêmes conditions que celles de l'étape a) ;
c) incuber les premier et second échantillons avec :

i) un anticorps sélectif pour NS2 dirigé contre un peptide consistant en SEQ ID NO : 6 ; ou
ii) un anticorps sélectif pour NS3 dirigé contre un peptide consistant en SEQ ID NO: 5;

dans des conditions appropriées pour réaliser la liaison de l'anticorps à NS2 ou NS3 ; et
d) déterminer la quantité d'anticorps liée au second échantillon et au premier échantillon produite dans l'étape c), où une diminution de la quantité d'anticorps dans le second échantillon comparée à celle du premier échantillon indique que le composé candidat peut être un inhibiteur de l'activité d'autoclivage de NS2/3.

**20.** Analyse selon la revendication 19 où de l'oxyde de lauryldiéthylamine (LDAO) est initialement présent dans ledit échantillon de NS2/3 pour empêcher l'autoclivage.

**21.** Analyse selon la revendication 20 où ledit LDAO est présent à une concentration de 1 % $\pm$ 10 %.

**22.** Analyse selon la revendication 20 ou 21 où, dans l'étape a), ledit échantillon est dilué de sorte que la concentration de LDAO est diminuée d'au moins 1,5 fois $\pm$ 10 % et est incubé en présence d'un agent d'activation pour induire l'autoclivage.

**23.** Analyse selon la revendication 22 où ledit agent d'activation est un détergent choisi dans le groupe consistant en : CHAPS, Triton X-100, NP-40 et le n-dodécyl-β-D-maltoside (DM).

**24.** Analyse selon la revendication 23 où ledit détergent jouant le rôle d'agent d'activation est présent au-delà de sa concentration micellaire critique (CMC).

**25.** Analyse selon l'une quelconque des revendications 19 à 24 où, dans l'étape a), ladite protéase est incubée dans une solution comprenant en outre du glycérol pour augmenter l'autoclivage.

**26.** Analyse selon la revendication 25 où ledit glycérol est présent à une concentration de 20 % $\pm$ 10 %.

**27.** Analyse selon l'une quelconque des revendications 19 à 26 où ledit anticorps est un anticorps polyclonal ou un anticorps monoclonal.

**28.** Analyse selon l'une quelconque des revendications 19 à 27 où ledit anticorps est détecté au moyen d'un marqueur détectable.

**29.** Analyse selon la revendication 28 où ledit marqueur détectable est choisi dans le groupe consistant en : un marqueur fluorescent, un marqueur chimiluminescent, un marqueur colorimétrique, un marqueur enzymatique et un isotope radioactif.

**30.** Analyse selon la revendication 29 où ledit marqueur détectable est l'europium.

**31.** Analyse selon la revendication 28, 29 ou 30 où ledit marqueur détectable est lié directement audit anticorps.

**32.** Analyse pour sélectionner un composé candidat pour l'activité inhibant le clivage de NS2/3 dans un échantillon contenant une protéase NS2/3 en présence de 1 % + 10 % d'oxyde de lauryldiéthylamine (LDAO), l'analyse comprenant :

a) diluer un premier échantillon comprenant une protéase NS2/3 en présence d'un agent d'activation pour permettre à au moins une partie de NS2/3 d'être clivée pour produire des produits NS2 et NS3 clivés, en l'absence de composé candidat ;
a') diluer encore la protéase dans ledit premier échantillon pour stopper l'autoclivage ;
a") immobiliser le produit NS3 provenant de l'étape a') ;
b) soumettre un second échantillon comprenant une protéase NS2/3 en présence d'un composé candidat aux mêmes conditions que celles de l'étape a) ;
b') diluer la protéase dans ledit second échantillon pour obtenir les mêmes conditions que celles de l'étape a') ;
b") immobiliser le produit NS3, s'il existe, provenant de l'étape b') ;
c) incuber NS3 immobilisé provenant de l'étape a") avec un anticorps dirigé contre le peptide APITAYSQQT [SEQ ID NO : 5], ledit anticorps étant marqué directement ou indirectement avec un marqueur détectable ;
c') incuber NS3 immobilisé provenant de l'étape b") avec le même anticorps que dans l'étape c) ;
d) déterminer la quantité d'anticorps marqué immobilisé dans chacune des étapes c) et c'), où une diminution de la quantité d'anticorps marqué immobilisé dans l'étape c') par rapport à celle pour l'étape c) indique que le composé candidat peut être un inhibiteur de l'activité de clivage de NS2/3.

**33.** Analyse selon la revendication 32 où les étapes a') et a") peuvent être accomplies en succession, ou en succession inversée, ou ensemble dans la même étape, et où ladite dilution dudit échantillon dans l'étape a') peut être conduite par lavage dudit échantillon immobilisé.

**34.** Analyse selon la revendication 32 ou 33 où ledit anticorps est marqué avec l'europium.

**35.** Anticorps dirigé contre un peptide de SEQ ID NO : 5, ledit anticorps étant sélectif pour le produit clivé NS3 et n'ayant pas ou ayant peu de réactivité croisée avec le produit clivé NS2 et NS2/3 non clivée comme déterminé par un signal qui est au moins 2 fois plus élevé $\pm$ 10 % contre le produit clivé NS3 que contre le produit clivé NS2 et NS2/3 non clivée.

**36.** Anticorps dirigé contre un peptide de SEQ ID NO : 6, ledit anticorps étant sélectif pour le produit clivé NS2 et n'ayant pas ou ayant peu de réactivité croisée avec le produit clivé NS3 et NS2/3 non clivée comme déterminé par un signal qui est au moins 2 fois plus élevé $\pm$ 10 % contre le produit clivé NS2 que contre le produit clivé NS3 et NS2/3 non clivée.

## FIGURE 1A

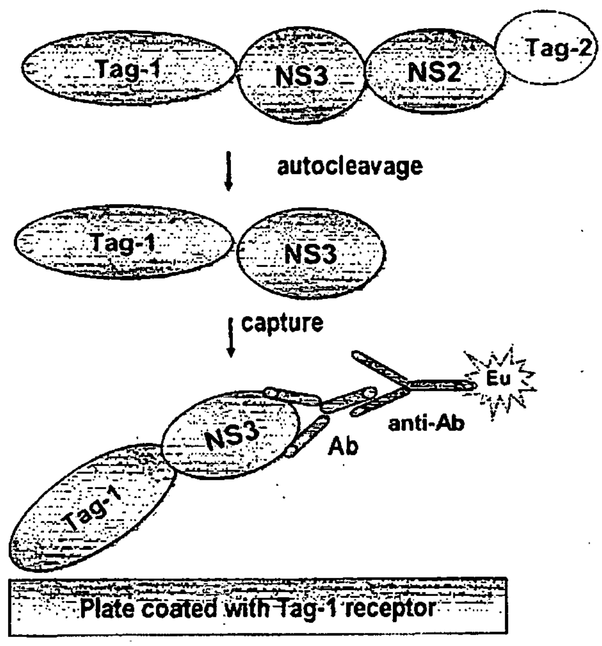


## FIGURE 1B

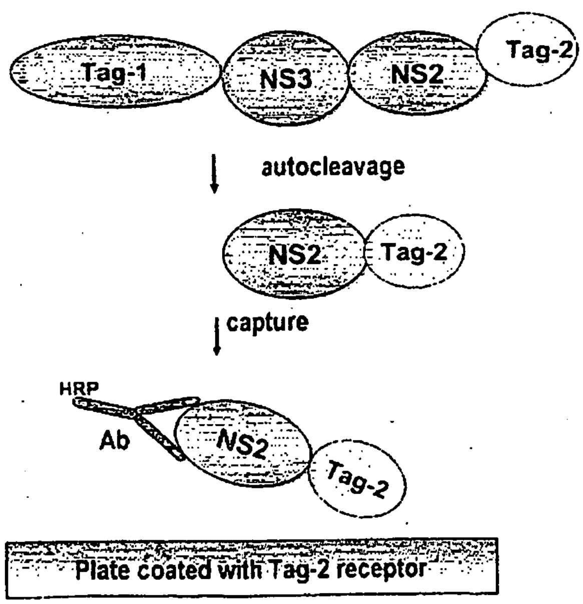

FIGURE 2A

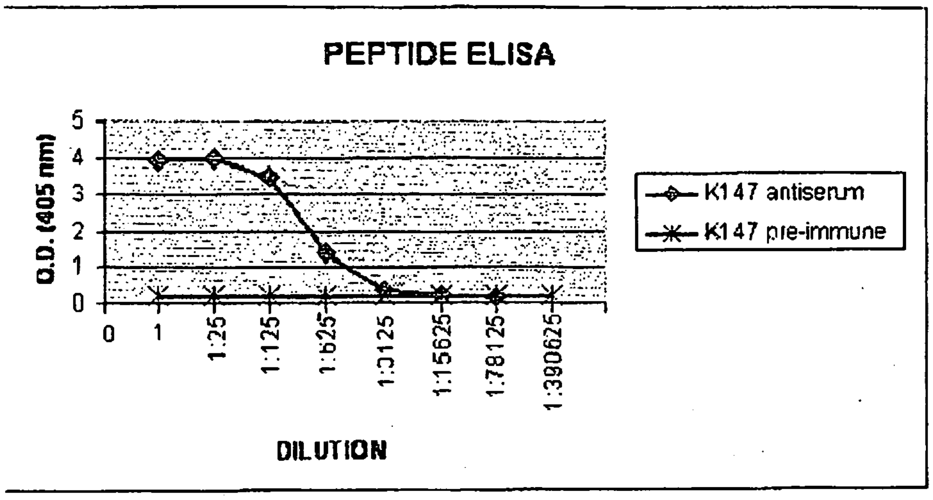


FIGURE 2B

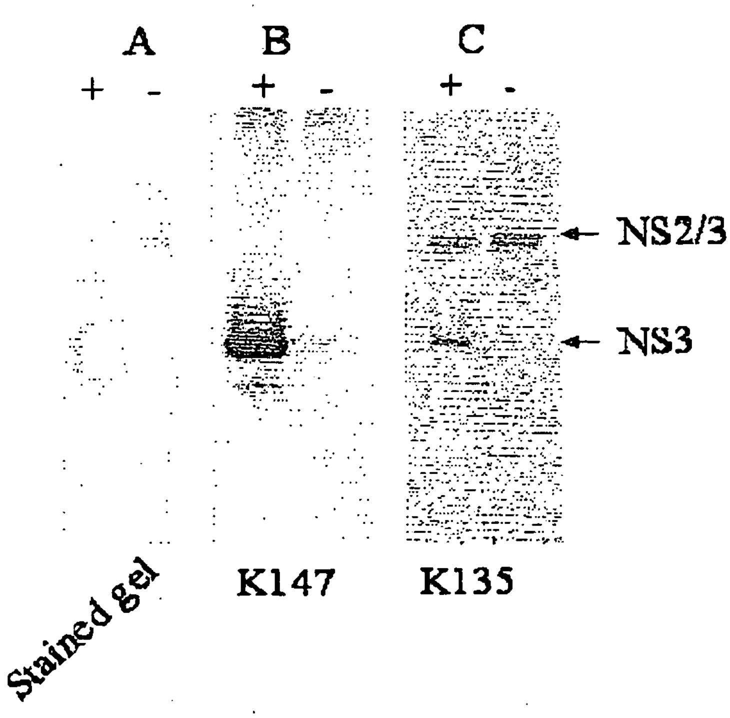

FIGURE 3

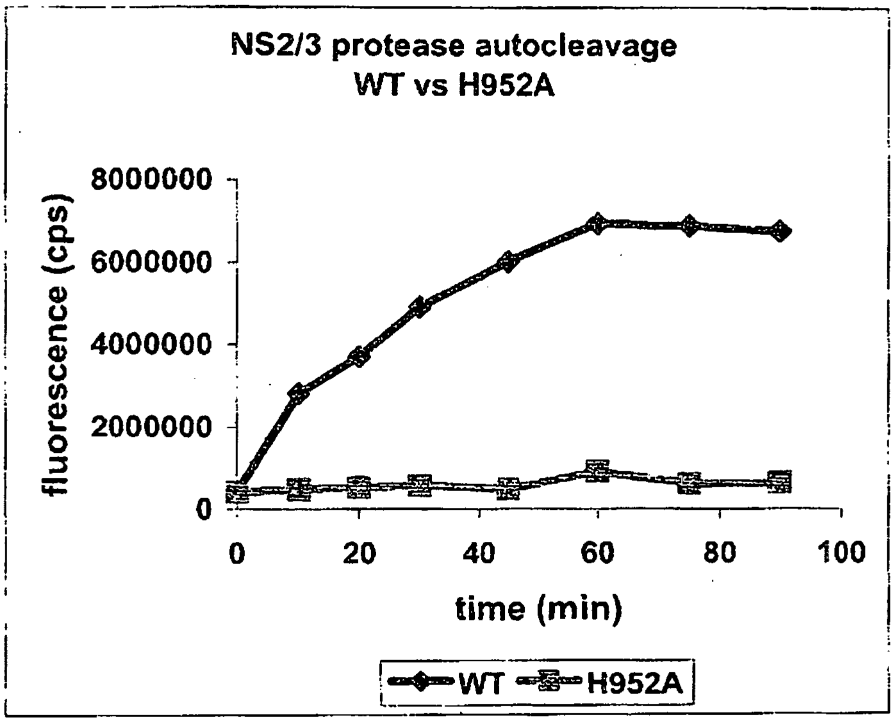


FIGURE 4

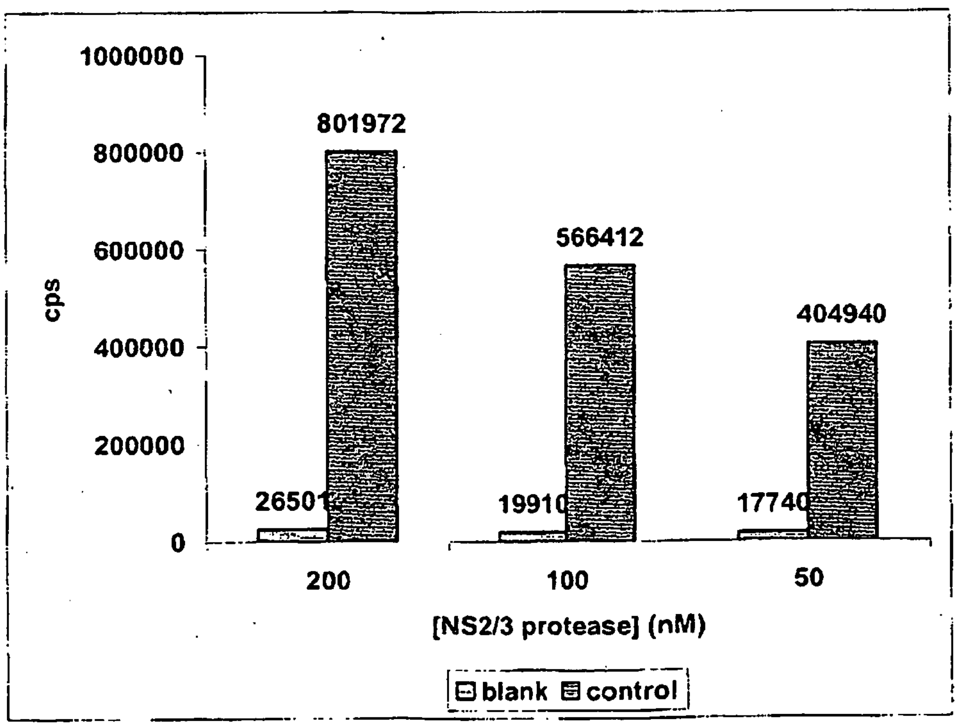

# FIGURE 5

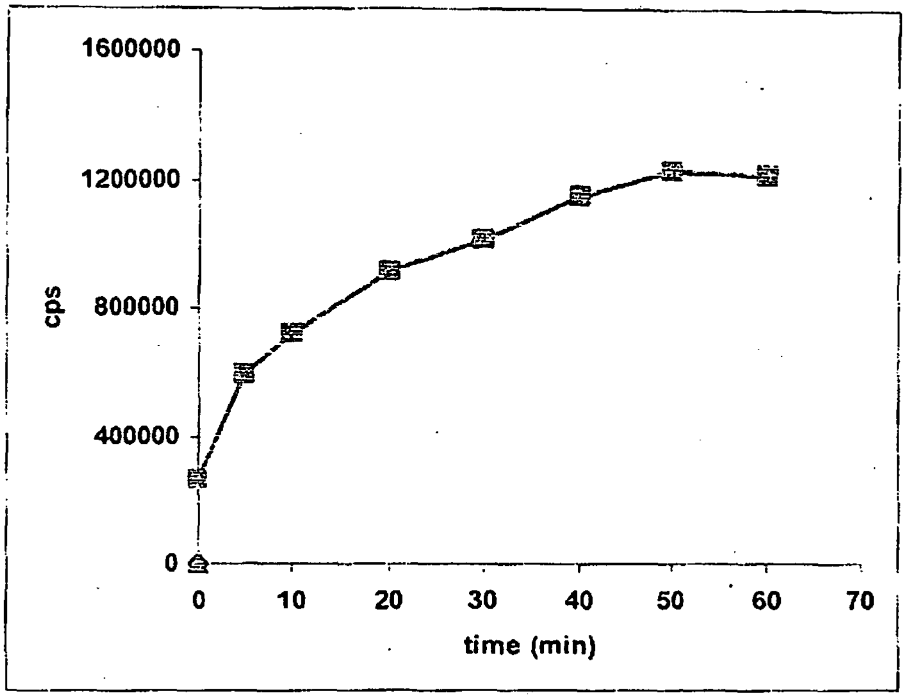


# FIGURE 6

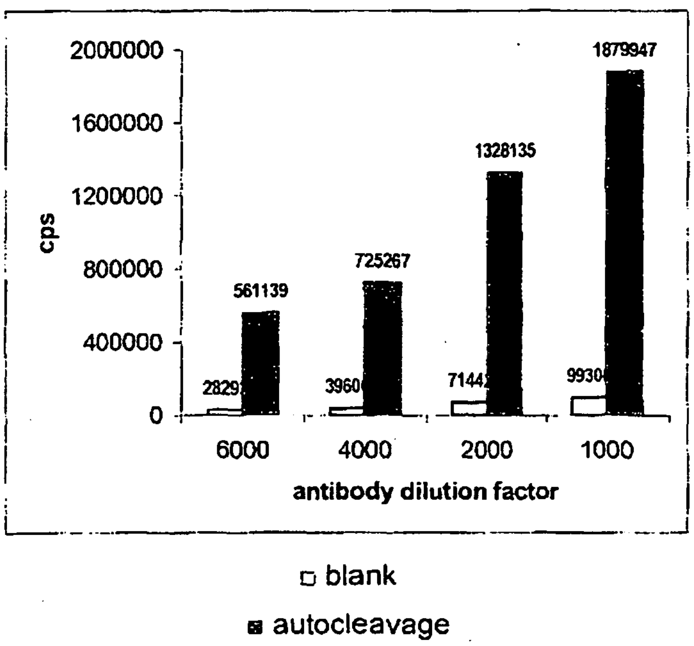

## FIGURE 7A

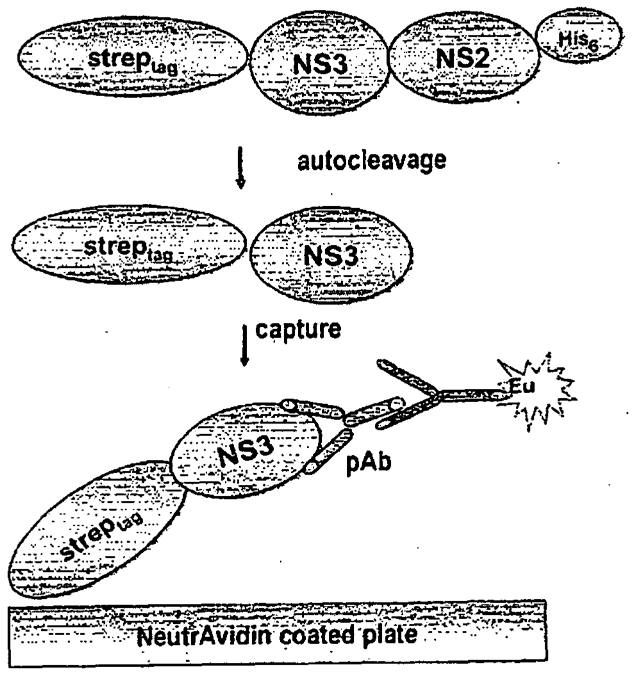


## FIGURE 7B

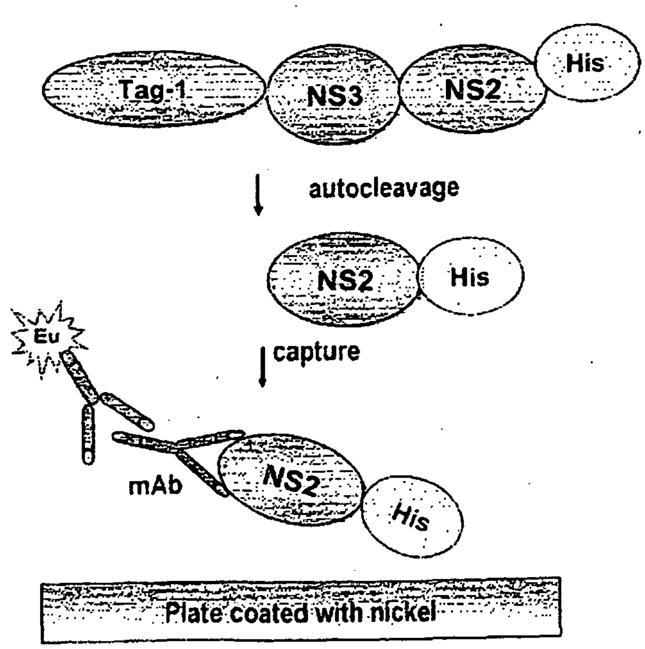

# FIGURE 8

## NS2/3 Protease Time-Resolved Fluorescence Assay
## Z' Determination

Plate 1

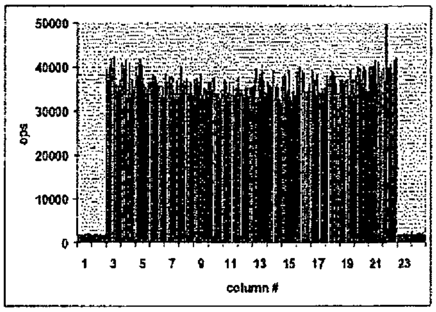


Plate 2

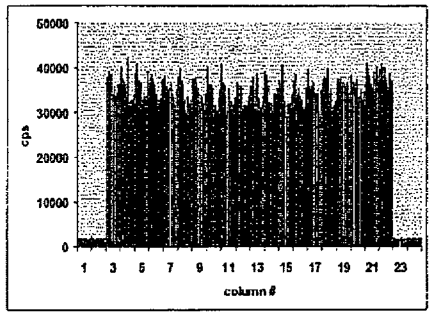


Plate 3

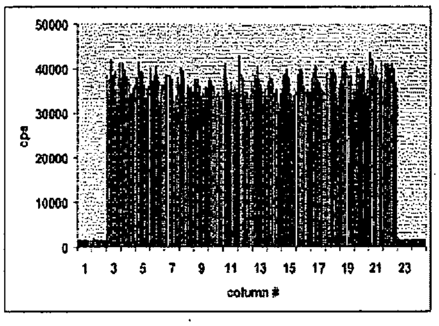


| Plate | Blank (cps) | Control (cps) | S/N | Z' |
|---|---|---|---|---|
| 1 | 1826 | 35942 | 19.7 | 0.75 |
| 2 | 1716 | 34324 | 20.0 | 0.70 |
| 3 | 1480 | 36375 | 24.6 | 0.73 |

# FIGURE 9

## NS2/3 Protease Time-Resolved Fluorescence Assay
## $IC_{50}$ Curve

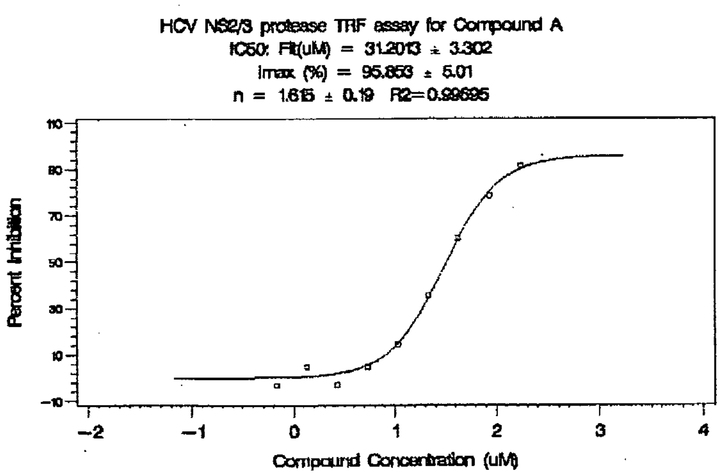

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0168818 A
- WO 0248375 A
- US 6815159 B
- WO 0168818 A2


### Non-patent literature cited in the description


- **SAMBROOK et al.** Molecular Cloning - A Laboratory Manual. Cold Spring Harbor Laboratory Press, 2001
- **AUSUBEL et al.** Current Protocols in Molecular Biology. Wiley, 1994
- **COLIGAN et al.** Current Protocols in Protein Science. John Wiley & Sons, Inc, 1995, vol. 1
- *Biochemistry,* 1972, vol. 11, 1726-1732
- Invitrogen- Molecular Probes Handbook - A Guide to Fluorescent Probes and Labeling Technology. 2005
- A guide to HTS Assay Development. April 2004
- **KOLYKHALOV, A.A. ; MIHALIK, K. ; FEINSTONE, S.M. ; C.M. RICE.** Hepatitis C virus-encoded enzymatic activities and conserved RNA elements in the 3'-nontranslated region are essential for virus replication in vivo. *J. Virol.,* 2000, vol. 74, 2046-2051
- **THIBEAULT, D. ; MAURICE, R. ; PILOTE, L. ; LAMARRE, D. ; PAUSE, A.** In vitro characterization of a purified NS2/3 protease variant of hepatitis C virus. *J. Biol. Chem.,* 2001, vol. 276 (49), 46678-46684
- **PALLAORO, M. ; LAHM, A. ; BIASIOL, G. ; BRUNETTI, M. ; NARDELLA, C. ; ORSATTI, L. ; BONELLI, F. ; ORRÙ, S ; NARJES, F. ; STEINKÜHLER, C.** Characterization of the hepatitis C virus NS2/3 processing reaction by using a purified precursor protein. *J. Virol.,* 2001, vol. 75, 9939-9946
- **WAUGH, D.S.** Making the most of affinity tags. *Trends Biotechnology,* 2005, vol. 23, 316-320
- **ZHANG J.-H. ; CHUNG, T.D.Y. ; OLDENBURG K.R.** A Simple Statistical Parameter for Use in Evaluation and Validation of High Throughput Screening. *J. of Biomol. Screening,* 1999, vol. 4 (2), 67-73